# EUROPEAN PATENT SPECIFICATION

(11) **EP 3 629 021 B1**
(45) Date of publication and mention of the grant of the patent: **25.11.2026**
(21) Application number: 19195605.1
(22) Date of filing: 05.09.2019
(51) Int. Cl.: G01N 33/564, G01N 33/68

(54) **DIAGNOSIS OF A NEUROAUTOIMMUNE DISEASE**
DIAGNOSE EINER NEUROAUTOIMMUNKRANKHEIT
DIAGNOSTIC D'UNE MALADIE NEURO-AUTOIMMUNE

(30) Priority: 26.09.2018 EP 18196867
(43) Date of publication of application: 01.04.2020
(73) Proprietor: EUROIMMUN Medizinische Labordiagnostika AG, 23560 Lübeck (DE)
(72) Inventor: SCHARF, Madeleine, 23923 Selmsdorf (DE); KOMOROWSKI, Lars, 23909 Ratzeburg (DE); MISKE, Ramona, 23564 Luebeck (DE); HAHN, Stefanie, 23858 Rheinfeld (DE); DENNO, Yvonne, 23558 Luebeck (DE); PROBST, Christian, 23909 Ratzeburg (DE); BENKHADRA, Farid, 1210 Luxembourg (LU)

(56) References cited:
- EP-A1- 2 952 898
- WO-A2-2018/060766
- JP-A- 2002 272 478
- T. BISOGNO: "Cloning of the first sn1-DAG lipases points to the spatial and temporal regulation of endocannabinoid signaling in the brain", THE JOURNAL OF CELL BIOLOGY, vol. 163, no. 3, 3 November 2003 (2003-11-03), pages 463 - 468, XP055075399, ISSN: 0021-9525, DOI: 10.1083/jcb.200305129
- SUÃ REZ J ET AL: "Distribution of diacylglycerol lipase alpha, an endocannabinoid synthesizing enzyme, in the rat forebrain", NEUROSCIENCE, NEW YORK, NY, US, vol. 192, 20 June 2011 (2011-06-20), pages 112 - 131, XP028279380, ISSN: 0306-4522, [retrieved on 20110626], DOI: 10.1016/J.NEUROSCIENCE.2011.06.062
- PACHER P ET AL: "Is lipid signaling through cannabinoid 2 receptors part of a protective system?", PROGRESS IN LIPID RESEARCH, PERGAMON PRESS, PARIS, FR, vol. 50, no. 2, 26 January 2011 (2011-01-26), pages 193 - 211, XP028166400, ISSN: 0163-7827, [retrieved on 20110202], DOI: 10.1016/J.PLIPRES.2011.01.001
- STOYAN POPKIROV ET AL: "Rho-associated protein kinase 2 (ROCK2): a new target of autoimmunity in paraneoplastic encephalitis", ACTA NEUROPATHOLOGICA COMMUNICATIONS, BIOMED CENTRAL LTD, LONDON, UK, vol. 5, no. 1, 29 May 2017 (2017-05-29), pages 1 - 10, XP021245512, DOI: 10.1186/S40478-017-0447-3

## Description

The present invention relates *inter alia* to a method for diagnosing a disease comprising the step detecting in a sample from a patient an autoantibody binding to DAGLA, a use of a polypeptide comprising DAGLA or a variant thereof for the diagnosis of a disease, an isolated human autoantibody binding to DAGLA, a complex comprising the autoantibody and the polypeptide, a method for isolating a human autoantibody binding DAGLA and a kit for the diagnosis of a disease comprising a polypeptide or a medical device comprising DAGLA or a variant thereof.

Developing diagnostic systems for neurological diseases is a continuing challenge in biomedical science, not in the least because many symptoms encountered may be accounted for by a huge variety of causes including genetically-inherited diseases, drug abuse, malnutrition, infection, injury, psychiatric illness, immunological defects and cancer.

Since a neurological disease is rarely associated with a unique characteristic pattern of clinical symptoms, it is often difficult to provide a reliable diagnosis solely based on the observation and examination of the patients affected or their medical history.

The importance of an early diagnosis cannot be overemphasized. Many neurological disorders, most prominently Alzheimer's and Parkinson's diseases as well as Multiple Sclerosis, cannot be cured, but drugs are available that may be used to slow down their progression. In addition, certain rare types of cancer are associated with neurological symptoms. The earlier the diagnosis, the better the chances to exploit the spectrum of available therapies to the full benefit of the patient.

This holds all the more true in the case of neurological diseases associated with autoantibodies. In some cases, the link between a specific detectable autoantibody and a condition is sufficiently strong to allow for an immediate diagnosis.

But even if it is not, the detection of autoantibodies may point the physician in charge to therapeutic means that may be used to ameliorate the patient's condition. There is a variety of widely used immunosuppressants that may be used regardless of the nature of the autoantibody's target. Alternatively, apheresis may be used to remove autoantibodies from the patient's blood. In many cases, patients went on to lead a normal life following early diagnosis and treatment of a neurological autoimmune disease.

Diagnostic assays based on the detection of autoantibodies may also corroborate the diagnosis of diseases other than those associated with autoantibodies. If it turns out that a blood sample is devoid of specific autoantibodies, this is likely to help the physician in charge exclude a range of possibilities and thus narrow down the spectrum of plausible conditions.

Examples of neurological conditions coinciding with the emergence of autoantibodies include Neuromyelitis optica, a disease characterized by loss of visual perception and spinal cord function, and anti-NMDA receptor encephalitis, which is associated with autonomic dysfunction, hypoventilation, cerebellar ataxia, hemiparesis, loss of consciousness, or catatonia. Whilst the involvement of autoantibodies and the nature of these conditions as such was previously poorly understood, many of this disease can now be diagnosed and treated efficiently owing to the availability of assays based on the detection of autoantibodies.

Therefore, it is paramount that new approaches be developed to distinguish neurological conditions associated with autoantibodies from those that are not.

EP2952898A1 and Popkirov et al., 2017 (doi: 10.1186/S40478-017-0447-3) disclose methods for diagnosing a (neurological) disease comprising detecting in a sample an autoantibody binding to an enzyme. WO 2018/060766 mentions a method comprising measuring the expression level of DAGLA

JP 2002 272478 mentions DAGLA antibodies produced by immunizing non-human mammals such as rabbits, rats and mice.

Suárez et al. (2011) Neuroscience 192:112-131 studies the neuronal distribution of DAGLA in the rat forebrain.

The present invention relates to autoantibodies to DAGLA and diagnostic assays based on their detection. As far as the inventors are aware, the existence of autoantibodies to DAGLA, let alone their diagnostic usefulness, has not yet been reported in the state of the art. A number of companies have commercialized recombinant antibodies binding to DAGLA, for example LifeSpan Biosciences, Inc and Sigma.

The problem underlying the present invention is to provide novel reagents, devices and methods that may be used to support the diagnosis of an autoimmune disease, preferably an autoimmune disease of the nervous system or associated with a neurological disease or neurological symptoms, more preferably selected from the group comprising PNS, cerebellitis, epilepsy and sclerosis.

Another problem underlying the present invention is to provide novel reagents, devices and methods that may be used to distinguish autoimmune diseases, in particular neurological autoimmune diseases, more preferably selected from the group comprising PNS, cerebellitis, epilepsy, ataxia, polyneuropathy and/or polyradiculopathy, and sclerosis, from diseases other than autoimmune diseases, for example from infections associated with neurological symptoms, not in the least to determine the most promising treatment regimen, more specifically whether or not an immunosuppressive treatment is adequate.

The problem underlying the present invention is solved by the subject-matter of the attached independent and dependent claims.

In one aspect, the problem is solved by a method for diagnosing a disease comprising the step detecting in a sample from a patient an autoantibody binding to DAGLA.

Further described herein is an immobilized polypeptide comprising DAGLA or a variant thereof.

In another aspect, the problem is solved by a use of a polypeptide comprising DAGLA or a variant thereof for the diagnosis of a disease, preferably comprising the step detecting in a sample an autoantibody binding to DAGLA.

Further described herein is a polypeptide comprising DAGLA or a variant thereof for use in a treatment of a disease.

In another aspect, the problem is solved by an isolated autoantibody to DAGLA.

Further described herein is a use of the autoantibody according to the present invention for the diagnosis of a disease.

In another aspect, the problem is solved by a method for isolating an autoantibody binding to DAGLA, comprising the steps
a) contacting a sample comprising the autoantibody with a polypeptide comprising DAGLA or a variant thereof under conditions compatible with formation of a complex, wherein said autoantibody binds to said polypeptide,
b) isolating the complex formed in step a),
c1) detecting the complex formed in step a) or c2) dissociating the complex isolated in step b) and separating the autoantibody from the polypeptide.

Further described herein is a pharmaceutical composition or medical device, preferably diagnostic device, comprising a polypeptide comprising DAGLA or a variant thereof.

In another aspect the problem is solved by a kit for the diagnosis of a disease, which kit comprises a polypeptide comprising DAGLA or a variant thereof or a medical device comprising a polypeptide comprising DAGLA or a variant thereof,
wherein the kit comprises in addition a means for detecting a complex comprising the polypeptide and an autoantibody binding to DAGLA,
wherein preferably the kit further comprises a positive control comprising DAGLA or a variant thereof or a negative control.

Further described herein is a use of a polypeptide comprising DAGLA or a variant thereof or an autoantibody binding to DAGLA or a medical device comprising a polypeptide comprising DAGLA or a variant thereof for the manufacture of a kit or medical device, preferably diagnostic device, for the diagnosis of a disease.

In a preferred embodiment, the disease is PNS, preferably associated with a condition selected from the group comprising cerebellitis, epilepsy and sclerosis. In another preferred embodiment, the disease is selected from the group comprising PNS, cerebellitis, epilepsy, sclerosis and a tumor, more preferably PNS, cerebellitis, epilepsy and sclerosis. In a preferred embodiment, the disease is a cancer, preferably from the group comprising a thoracic cancer, more preferably lung cancer or thymus cancer, most preferably from the group comprising small cell lung cancer, carcinoid and non-small cell lung cancer; a breast, gynecologic and testicular cancer; and hematologic malignancies and solid tumors, preferably from the group comprising lymphoma, leukemia, melanoma, urinary tract cancers, gastrointestinal cancers, colon cancer, gastric and esophageal cancers, head and neck cancers, sarcoma and histiocytosis.

In a preferred embodiment, the sample is a bodily fluid comprising antibodies, preferably selected from the group comprising whole blood, plasma, serum, cerebrospinal fluid and saliva.

In a preferred embodiment, the autoantibody or complex is detected using a technique selected from the group comprising immunodiffusion techniques, immunoelectrophoretic techniques, light scattering immunoassays, agglutination techniques, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

In a preferred embodiment, the medical device is selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

The present invention is based on the inventors' surprising finding that an autoantibody to DAGLA exists and may be detected in samples from a number of patients suffering from neurological conditions, but not in samples obtained from healthy subjects.

Furthermore, the present invention is based on the inventors' surprising finding that the novel neurological disease may be diagnosed by way of detection of an autoantibody to DAGLA. Without wishing to be bound to any theory, the presence of such autoantibodies suggests that the function of DAGLA and/or downstream effectors is impaired in patients having such autoantibodies to the effect that neurological symptoms occur.

DAGLA (diacylglycerol lipase alpha, 120 kDa, 1.042 amino acids) is, together with DAGL-beta, a member of the DAG lipase (DAGL) family.

DAGLs are responsible for catalyzing the hydrolysis of diacylglycerol (DAG), one of the most studied second messenger in cells, to 2-arachidonoyl-glycerol (2-AG), the most abundant ligand for the endocannabinoid receptors (eCB) in the body (Keimpema, E. et al. (2013) Diacylglycerol lipase α manipulation reveals developmental roles for intercellular endocannabinoid signaling. Sci Rep. 3:2093/doi: 10.1038/srep02093). DAGL-dependent eCB signalling regulates axonal growth and guidance during development and is required for the generation and migration of new neurons in the adult brain (Williams, E.J. et al. (1994) The production of arachidonic acid can account for calcium channel activation in the second messenger pathway underlying neurite outgrowth stimulated by NCAM, N-cadherin, and L1. J. Neurochem. 62:1231-1234/doi:10.1046/j.1471-4159.1994.62031231.x). Beyond the encannabinoid system DAGLs influence the level of the essential lipid arachidonic acid via the synthesis of 2-AG in the brain and other organs (Reisenberg, M. et al. (2012) The diacylglycerol lipases: structure, regulation and roles in and beyond endocannabinoid signalling. Phil. Trans. R. Soc. B: 367, 3264-3275/doi:10.1098/rstb.2011.0387).

2-AG is mainly synthesized by DAGLA isoform whose expression correlates strongly with the total biosynthesis and release of 2-AG. The expression pattern of DAGLA changes from axonal tracts in the embryo to the dendritic fields and proliferating neural stem cells in the subventricular zone in the adult (Bisogno, T. et al. (2003) Cloning of the first sn1-DAG lipases points to the spatial and temporal regulation of endocannabinoid signaling in the brain. J Cell Biol. 163(3):463-8/doi/10.1083/jcb.200305129).

Genetic defects in DAGLA are potentially associated with the development of spinocerebellar ataxia type 20 (SCA20) in humans (Knight, M.A. et al. (2008) A duplication at chromosome 11q12.2-11q12.3 is associated with spinocerebellar ataxia type 20. Hum Mol Genet. 17(24):3847-3853. doi:10.1093/hmg/ddn283). Spinocerebellar ataxia is a clinically and genetically heterogeneous group of cerebellar disorders. Patients show progressive incoordination of gait and often poor coordination of hands, speech and eye movements, due to degeneration of the cerebellum with variable involvement of the brainstem and spinal cord (Knight, M.A. et al. (2004) Dominantly inherited ataxia and dysphonia with dentate calcification: spinocerebellar ataxia type 20. Brain. 127: 1172-1181/doi: 10.1093/brain/awh139).

DAGLA consists of a short cytoplasmic N-terminal sequence leading to a 4 transmembrane (4TM) helix domain, followed by a canonical alpha/beta hydrolase domain that harbors the catalytic domain, followed by a long C-terminal tail domain. The 4TM domain is conserved in the 19 AA N-terminal sequence. The 4TMs are separated by short unconcerned loops and two extracellular transmembrane domains represent potential sites for glycosylation.

Functionally, it is assumed that DAGLA facilitates the packing of enzymes at the membrane and acts as docking sites for other proteins in a functional complex and forms a channel that regulates DAG access to the catalytic domain and release of 2-AG from cells afterwards. The catalytic domain consists of in total 8 mutually hydrogen-bonded beta-sheet strands linked mostly by alpha-helices. Characteristically, it has a catalytic serine (AA 472) and aspartic acid (AA 524) and histidine (AA 650), the so called catalytic triad (Baggelaar, M.P. et al. (2013) Development of an Activity-Based Probe and In Silico Design Reveal Highly Selective Inhibitors for Diacylglycerol Lipase-alpha in Brain. Angw. Chem. Int. Ed. 52:1-6/doi:10.1002/anie.201306295). The catalytic action of DAGLA requires Ca2+ as an essential cofactor. Further, the catalytic domain includes a regulatory loop (appr. 50-60 AA long, between 7th and 8th canonical beta-sheet), which harbors a conserved 10 AA poly-proline signature motif (PLYPPGRIIH). This might function as cap or lid to shield hydrophobic catalytic cavity from water (Miled, N. et al. (2003) Importance of the lid and cap domains for the catalytic activity of gastric lipases. Comp Biochem Physiol B Biochem Mol Biol. 136(1):131-8/ doi:10.1016/S1096-4959(03)00183-0). The C-terminus of DAGLA is the most characteristic structural difference between the alpha and beta isoform. DAGLA but not DAGL-beta shows a long intracellular sequence. This DAGLA specific tail does not directly contribute to catalytic activity. Though, the integrated consensus motif (PPxxF) has been shown to bind the coiled-coil domain of Homer proteins (e.g. in mGluR interaction).

The present invention relates to a polypeptide comprising a mammalian, preferably human polypeptide selected from DAGLA or antigenic variants reactive to autoantibodies binding to DAGLA. Mammalian DAGLA includes homologues from human, monkey, mouse, rat, rabbit, guinea pig or pig, preferably human.

In a more preferred embodiment, DAGLA is the polypeptide encoded by SEQ ID NO4, Q9Y4D2 (alpha) (identical to SEQ ID NO4), NP_631918.3 (beta isoform 1) NP_001136408.1 (beta isoform 2), preferably Q9Y4D2. The cDNA consisting of the nucleic acid encoding SEQ ID NO4 is SEQ ID NO1. Throughout this application, any data base codes cited refers to the Uniprot data base, more specifically the version on the filing date of this application or its earliest priority application.

The teachings of the present invention may not only be carried out using polypeptides, in particular a polypeptide comprising the native sequence of a polypeptide such as DAGLA or nucleic acids having the exact sequences referred to in this application explicitly, for example by function, name, sequence or accession number, or implicitly, but also using variants of such polypeptides or nucleic acids.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 6, 7, 8, 10, 12, 15, 20, 25, 50, 75, 100, 150 or 200 successive amino acids of the original sequence or a variant thereof. The total length of the variant may be at least 6, 7, 8, 9, 10, 11, 12, 20, 25, 30, 40, 50, 60, 70, 80, 90, 100, 200, 300, 400, 500, 750, 1000 or more amino acids.

The term "variant" relates not only to at least one fragment, but also to a polypeptide or a fragment thereof comprising amino acid sequences that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability of an antigen to bind to an (auto)antibody, or the fold or structure of the polypeptide are deleted or substituted and/or one or more such essential amino acids are replaced in a conservative manner and/or amino acids are added such that the biological activity of the polypeptide is preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default settings. Preferred variants or fragments having such biological activity are SEQ ID NO26 and SEQ ID NO28 and the His-tagged versions of them.

In a preferred embodiment, the variant is a linear, non-folded polypeptide, which is optionally denatured.

In a preferred embodiment, the polypeptide and variants thereof may, in addition, comprise chemical modifications, for example isotopic labels or covalent modifications such as glycosylation, phosphorylation, acetylation, decarboxylation, citrullination, methylation, hydroxylation and the like. The person skilled in the art is familiar with methods to modify polypeptides. Any modification is designed such that it does not abolish the biological activity of the variant.

Moreover, variants may also be generated by fusion with other known polypeptides or variants thereof and comprise active portions or domains, preferably having a sequence identity of at least 70, 75, 80, 85, 90, 92, 94, 95, 96, 97, 98 or 99 % when aligned with the active portion of the reference sequence, wherein the term "active portion", as used herein, refers to an amino acid sequence, which is less than the full length amino acid sequence or, in the case of a nucleic acid sequence, codes for less than the full length amino acid sequence, respectively, and/or is a variant of the natural sequence, but retains at least some of the biological activity.

In a preferred embodiment, the term "variant" of a nucleic acid comprises nucleic acids the complementary strand of which hybridizes, preferably under stringent conditions, to the reference or wild type nucleic acid. Stringency of hybridization reactions is readily determinable by one of ordinary skilled in the art, and in general is an empirical calculation dependent on probe length, washing temperature and salt concentration. In general longer probes require higher temperatures for proper annealing, while shorter probes less so. Hybridization generally depends on the ability of denatured DNA to reanneal to complementary strands present in an environment below their melting temperature: The higher the degree of desired homology between the probe and hybridizable sequence, the higher the relative temperature which may be used. As a result, higher relative temperatures would tend to make the reaction conditions more stringent, while lower temperature less so. For additional details and explanation of stringency of hybridization reactions, see Ausubel, F. M. (1995), Current Protocols in Molecular Biology. John Wiley & Sons, Inc. Moreover, the person skilled in the art may follow the instructions given in the manual Boehringer Mannheim GmbH (1993) The DIG System Users Guide for Filter Hybridization, Boehringer Mannheim GmbH, Mannheim, Germany and in Liebl, W., Ehrmann, M., Ludwig, W., and Schleifer, K. H. (1991) International Journal of Systematic Bacteriology 41: 255-260 on how to identify DNA sequences by means of hybridization. In a preferred embodiment, stringent conditions are applied for any hybridization, i.e. hybridization occurs only if the probe is 70 % or more identical to the target sequence. Probes having a lower degree of identity with respect to the target sequence may hybridize, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C - 68°C, approximately 56°C - 68°C, approximately 58°C - 68°C, approximately 60°C - 68°C, approximately 62°C - 68°C, approximately 64°C - 68°C, approximately 66°C - 68°C. In a particularly preferred embodiment, the temperature is approximately 64°C - 68°C or approximately 66°C - 68°C. It is possible to adjust the concentration of salt to 0.2 x SSC or even 0.1 x SSC. Nucleic acid sequences having a degree of identity with respect to the reference or wild type sequence of at least 70, 80, 90, 91, 92, 93, 94, 95, 96, 97, 98, 99 % may be isolated. In a preferred embodiment, the term variant of a nucleic acid sequence, as used herein, refers to any nucleic acid sequence that encodes the same amino acid sequence and variants thereof as the reference nucleic acid sequence, in line with the degeneracy of the genetic code.

The variant of the polypeptide has biological activity. In a preferred embodiment, such biological activity is the ability to bind specifically to an autoantibody binding to DAGLA, as found in a patient suffering from an autoimmune disease associated with such autoantibody, preferably associated with a neurological disease or condition such as PNS. For example, whether or not a variant of DAGLA has such biological activity may be checked by determining whether or not the variant of interest binds to an autoantibody from a sample of a patient which autoantibody binds to wild type DAGLA, preferably as determined by indirect immunofluorescence using primate cerebellum as described in the experimental section of this application.

Any polypeptide according to the present invention, when used to carry out the teachings of the present invention, may be provided in any form and at any degree of purification, from liquid samples, tissues or cells comprising said polypeptide in an endogenous form, more preferably cells overexpressing the polypeptide, crude or enriched lysates of such cells, to purified and/or isolated polypeptide which is optionally essentially pure. In a preferred embodiment, the polypeptide is a native polypeptide, wherein the term "native polypeptide", as used herein, refers to a folded polypeptide, more preferably to a folded polypeptide purified from tissues or cells, more preferably from mammalian cells or tissues, optionally from non-recombinant tissues or cells. In another preferred embodiment, the polypeptide is a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", "Purifying Challenging Proteins" (2009/2010), published by GE Healthcare Life Sciences, and in Burgess, R. R., Deutscher, M. P. (2009), Guide to Protein Purification). In a preferred embodiment, a polypeptide is pure if at least 60, 70, 80, 90, 95 or 99 percent of the polypeptide in the respective sample consists of said polypeptide as judged by SDS polyacrylamide gel electrophoresis followed by Coomassie blue staining and visual inspection.

If the inventive polypeptide is provided in the form of tissue, it is preferred that the tissue is mammalian tissue, for example human, rat, primate, donkey, mouse, goat, horse, sheep, pig or cow, more preferably brain tissue, most preferably cerebellum. If a cell lysate is used, it is preferred that the cell lysate comprises the membranes associated with the surface of the cell or is in fact a fraction enriched in membranes. If said polypeptide is provided in the form of a recombinant cell, it is preferred that the recombinant cell is a eukaryotic cell such as a yeast cell, more preferably a cell from a multicellular eukaryote such as a plant, mammal, frog or insect, most preferably from a mammal, for example rat, human, primate, donkey, mouse, goat, horse, sheep, pig or cow.

The polypeptide used to carry out the inventive teachings, including any variants, is preferably designed such that it comprises at least one epitope recognized by and/or binds specifically to the autoantibody binding to DAGLA. Any epitope is more preferably an epitope recognized by such an autoantibody only, by contrast to antibodies other than an autoantibody to DAGLA. In one embodiment, such polypeptide comprises a stretch of 6, 7, 8, 9, 10, 11, 12, 20, 25, 30, 40, 50, 60, 70, 80, 90, 100 or more, preferably at least 9 but no more than 16, consecutive amino acids from DAGLA. The person skilled in the art is familiar with guidelines used to design peptides having sufficient immunogenicity, for example those described in Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173. Briefly, it is desirable that the peptide meets as many as possible of the following requirements: (a) it has a high degree of hydrophilicity, (b) it comprises one or more residues selected from the group comprising aspartate, proline, tyrosine and phenylalanine, (c) is has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71. Suitable epitopes are present in SEQ ID NO 16 and SEQ ID NO 22.

The inventive polypeptide, when used according to the present invention, may be provided in any kind of conformation. For example, the polypeptide may be an essentially unfolded, a partially or a fully folded polypeptide. In a preferred embodiment, the polypeptide is folded in the sense that the epitopes essential for the binding to the inventive autoantibody, or the protein or variant thereof in its entirety, adopt the fold adopted by the native protein in its natural environment. The person skilled in the art is familiar with methods suitable to determine whether or not a polypeptide is folded and if it is, which structure it has, for example limited proteolysis, NMR spectroscopy, CD spectroscopy or X-ray crystallography (see for example Banaszak L. J. (2008), Foundations of Structural Biology, Academics Press, or Teng Q. (2013), Structural Biology: Practical Applications, Springer), preferably CD spectroscopy is used.

The inventive polypeptide may be a fusion protein which comprises amino acid sequences other than those taken from DAGLA, in particular a C-terminal or N-terminal tag, preferably a C-terminal tag, which is, in a preferred embodiment, as used herein, an additional sequence motif or polypeptide having a function that has some biological or physical function and may, for example, be used to purify, immobilize, precipitate or identify the inventive polypeptide. In a more preferred embodiment, the tag is a sequence or domain capable of binding specifically to a ligand, for example a tag selected from the group comprising His tags, thioredoxin, maltose binding protein, glutathione-S-transferase, a fluorescence tag, for example from the group comprising green fluorescent protein.

The inventive polypeptide may be an immobilized polypeptide. In a preferred embodiment, the term "immobilized", as used herein, refers to a molecule bound to a solid carrier insoluble in an aqueous solution, more preferably via a covalent bond, electrostatic interactions, encapsulation or entrapment, for example by denaturing a globular polypeptide in a gel, or via hydrophobic interactions, most preferably via one or more covalent bonds. Various suitable carriers, for example paper, polystyrene, metal, silicon or glass surfaces, microfluidic channels, membranes, beads such as magnetic beads, column chromatography media, biochips, polyacrylamide gels and the like have been described in the literature, for example in Kim, D., and Herr, A. E. (2013), Protein immobilization techniques for microfluidic assays, Biomicrofluidics 7(4), 041501. This way, the immobilized molecule, together with the insoluble carrier, may be separated from an aqueous solution in a straightforward manner, for example by filtration, centrifugation or decanting. An immobilized molecule may be immobilized in a reversible or irreversible manner. For example, the immobilization is reversible if the molecule interacts with the carrier via ionic interactions that can be masked by addition of a high concentration of salt or if the molecule is bound via a cleavable covalent bond such as a disulphide bridge which may be cleaved by addition of thiol-containing reagents. By contrast, the immobilization is irreversible if the molecule is tethered to the carrier via a covalent bond that cannot be cleaved in aqueous solution, for example a bond formed by reaction of an epoxide group and an amine group as frequently used to couple lysine side chains to affinity columns. The protein may be indirectly immobilized, for example by immobilizing an antibody or other entity having affinity to the molecule, followed by formation of a complex to the effect that the molecule-antibody complex is immobilized. Various ways to immobilize molecules are described in the literature, for example in Kim, D., Herr, and A. E. (2013), Protein immobilization techniques for microfluidic assays, Biomicrofluidics 7(4), 041501. In addition, various reagents and kits for immobilization reactions are commercially available, for example from Pierce Biotechnology.

It is essential that the sample used for the diagnosis in line with the detection of autoantibodies according to the present invention comprises antibodies, also referred to as immunoglobulins. Typically the sample of a bodily fluid comprises a representative set of the entirety of the subject's immunoglobulins. However, the sample, once provided, may be subjected to further processing which may include fractionation, centrifugation, enriching or isolating the entirety of immunoglobulins or any immunoglobulin class of the subject, which may affect the relative distribution of immunoglobulins of the various classes.

The reagents, devices, methods and uses described throughout this application may be used for the diagnosis of a disease. In a preferred embodiment, the disease is a neurological disease. In a more preferred embodiment, the term "neurological disease", as used herein, refers to any disease associated with a defect of the nervous system, in another preferred embodiment, the term "PNS", abbreviation of paraneoplastic neurological syndrome, as used herein, refers to a systemic disorder indirectly caused by the presence of a tumor, for example, as a result of the production release of substances such as hormones or cytokines not normally produced by the cell of origin of the tumor or are produced at increased concentration or the production and release of biologically active cells. Such systemic order may be revealed by various conditions comprising cerebellitis, epilepsy and sclerosis. Any manifestation of PNS indicates that the patient should be thoroughly examined for the presence of a tumor, although the tumor may be too small for detection.

Cerebellitis can have a variety of causes and be related to the following diseases, which may therefore be diagnosed or distinguished or differentiated from autoimmune cerebellitis using the present invention: Virus infection, for example with Coxsackie, Influenza, Varicella Zoster, Eppstein-Barr, Rotavirus or Parvovirus, bacterial infection, for example Mycobacterium pneumoniae or Cryptococcus, ataxia, autoimmune cerebellitis, for example associated with autoantibodies to Neurochrondrin or Yo, drug overdose, for example by methadone or opium, vaccine-induced cerebellitis.

Epilepsy can have a variety of causes and be related to the following diseases, which may therefore be diagnosed or distinguished or differentiated from autoimmune cerebellitis using the present invention: benign febrile convulsions of childhood, idiopathic/cryptogenic seizures, cerebral dysgenesis, symptomatic epilepsy, head trauma, stroke, vascular malformations, mass lesions, CNS infections, encephalitis, ceningitis, cysticercosis, HIV encephalopathy, hypoglycemia, hyponatremia, hyperolsmolar states, hypocalcemia, uremia, hepatic encephalopathy, parophyria, drug toxicity, drug withdrawal, global cerebral ischemia, hypertensive encephalopathy, eclampsia, hyperthermia.

Sclerosis can have a variety of causes and be related to the following diseases, which may therefore be diagnosed or distinguished or differentiated from autoimmune cerebellitis using the present invention: Lou Gehrig's disease, Atherosclerosis, nephrotic syndrome, Hippocampal sclerosis, Lichen sclerosus and Multiple sclerosis.

Therefore, the present invention may also be used for distinguishing an autoimmune disease from an infectious disease, in particular a neuronal autoimmune disease from an infectious disease. Detection of the autoantibody to DAGLA shows that the disease is an autoimmune disease.

In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a certain treatment, for example the administration of immunosuppressive drugs. In other words, the term "diagnosis" comprises not only diagnosing, but also prognosticating and/or monitoring the course of a disease or disorder. In many cases the mere detection, in other words determining whether or not detectable levels of the antibody are present in the sample, is sufficient for the diagnosis. If the autoantibody can be detected, this will be information instrumental for the clinician's diagnosis and indicates an increased likelihood that the patient suffers from a disease. In a preferred embodiment, the autoantibody is deemed detectable if it can be detected using one or more methods selected from the group comprising immunoprecipitation, indirect immunofluorescence, ELISA or line blot, preferably indirect immunofluorescence. In a preferred embodiment, the relative concentration of the antibody in the serum, compared to the level that may be found in the average healthy subject, may be determined. While in many cases it may be sufficient to determine whether or not autoantibodies are present or detectable in the sample, the method carried out to obtain information instrumental for the diagnosis may involve determining whether the concentration is at least 2, preferably 5, 10, 20, 25, 50, 100, 200, 500, 1000, 10000 or 100000 times higher than the concentration found in the average healthy subject. In a preferred embodiment, the relative concentration of the autoantibody is determined using one or more methods selected from the group comprising semi-quantitative immunoprecipitation, semi-quantitative semi-quantitative indirect immunofluorescence, ELISA or semi-quantitative line blot, preferably ELISA. Experimental details are as described in the experimental section of this application or as in text books or practical manuals as available at the priority date of this application. Many assays may be carried out in a competitive format, wherein DAGLA or a variant thereof is bound to a first antibody, which is replaced by a second antibody. For example, the first antibody may be the autoantibody to DAGLA and the second antibody may be a recombinant antibody, preferably labeled with a detectable label.

The person skilled in the art will appreciate that a clinician does usually not conclude whether or not the patient suffers or is likely to suffer from a disease, condition or disorders solely on the basis of a single diagnostic parameter, but needs to take into account other aspects, for example the presence of other autoantibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred embodiment, the meaning of any symptoms or diseases referred to throughout this application is in line with the person skilled in the art's understanding as of the filing date or, preferably, earliest priority date of this application as evidenced by text books and scientific publications. It should be mentioned that the inventive methods or uses or products, taken alone, cannot be used to arrive at a definite, final diagnosis.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", i. e. a systematic diagnostic procedure considering the likelihood of a range of possible conditions on the basis of a range of diagnostic parameters. Consequently, the inventive method, polypeptide or use, optionally for determining whether a patient suffers from the a disease, may comprise obtaining a sample from a patient, preferably a human patient, determining whether an autoantibody binding to DAGLA is present in said sample, wherein said determining is performed by contacting the sample with the inventive polypeptide and detecting whether binding occurs between said polypeptide and said autoantibody, preferably using a labeled secondary antibody, wherein said autoantibody binds to said polypeptide if present in the sample, and diagnosing the patient as suffering or being more likely to suffer from said neurological disorder if the autoantibody was determined to be present in the sample.

The term "diagnosis" may also refer to a method or agent used to distinguish between two or more conditions associated with similar or identical symptoms.

The term "diagnosis" may also refer to a method or agent used to choose the most promising treatment regime for a patient. In other words, the method or agent may relate to selecting a treatment regimen for a subject. For example, the detection of autoantibodies may indicate that an immunosuppressive therapy is to be selected, which may include administrating to the patient one or more immunosuppressive drugs.

The present invention relates to a complex comprising an antibody, preferably autoantibody, binding to the inventive polypeptide. Such a complex may be used or detected as part of a method for diagnosing a disease. A liquid sample comprising antibodies from a subject may be used to practice the method if an autoantibody to DAGLA is to be detected. Such a liquid sample may be any bodily fluid comprising a representative set of antibodies from the subject, preferably a sample comprising antibodies of an immunoglobulin class from the subject selected from the group comprising IgG, IgA and IgM class antibodies, preferably IgG, more preferably IgG1 and IgG2, more preferably IgG1. For example, a sample may be cerebrospinal fluid (CSF), blood or blood serum, lymph, interstitial fluid and is preferably serum or CSF, more preferably serum. It is preferably an *ex vivo* sample.

The step contacting a liquid sample comprising antibodies with the inventive polypeptide(s) may be carried out by incubating an immobilized form of said polypeptide(s) in the presence of the sample comprising antibodies under conditions that are compatible with the formation of the complex comprising the respective polypeptide and an antibody, preferably an autoantibody, binding to the inventive polypeptide. The liquid sample, then depleted of antibodies binding to the inventive polypeptide(s) may be removed subsequently, followed by one or more washing steps. Finally the complex comprising the antibody or antibodies and the polypeptide(s) may be detected. In a preferred embodiment, the term "conditions compatible with the formation of the complex" are conditions that allow for the specific antigen-antibody interactions to build up the complex comprising the polypeptide and the antibody. In a preferred embodiment such conditions may comprise incubating the polypeptide in sample diluted 1:100 in PBS buffer for 30 minutes at 25 °C.

In a preferred embodiment, the term "autoantibody", as used herein, refers to an antibody binding specifically to an endogenous molecule of the animal, preferably mammal, more preferably human, which produces said autoantibody, wherein the level of such antibody is more preferably elevated compared the average of any other antibodies binding specifically to such an endogenous molecule. In a most preferred embodiment, the autoantibody is an autoantibody binding to DAGLA. The autoantibody may have the sequence of an antibody's constant regions from the animal, preferably human, making it, but the variable region is able to bind specifically to the endogenous molecule of the animal, more specifically DAGLA. In a preferred embodiment, the autoantibody is isolated and/or purified from a sample, preferably tissue, serum, plasma, blood or CSF from the animal, preferably human. The autoantibody is a polyclonal, native antibody from the animal rather than a synthetic or recombinant antibody. In a preferred embodiment, the autoantibody is an antibody binding specifically to SEQ ID NO 26 or SEQ ID NO 28, preferably SEQ ID NO28.

The method according to the present invention is preferably an *in vitro* method.

In a preferred embodiment, the detection of the complex for the prognosis, diagnosis, methods or test kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassay, enzyme immunoassays, preferably ELISA, chemiluminescence immunoassays, and immunofluorescence, preferably indirect immune-fluorescence techniques. The person skilled in the art is familiar with these methods, which are also described in the state of the art, for example in Zane, H. D. (2001), Immunology - Theoretical & Practical Concepts in Laboratory Medicine, W. B. Saunders Company, in Chapter 14.

Alternatively, a sample comprising tissue comprising the inventive polypeptide rather than a liquid sample may be used. The tissue sample is preferably from a tissue expressing endogenous DAGLA, preferably at an increased level compared to the average tissue in the respective organism's, preferably human body. Such a sample, which may be in the form of a tissue section fixed on a carrier, for example a glass slide for microscopic analysis, may then be contacted with the inventive antibody, preferably autoantibody, binding to the inventive polypeptide. The antibody is preferably labeled to allow for distinction from endogenous antibodies binding to the inventive polypeptide, so that newly formed complexes may be detected and, optionally, quantified. If the amount of complexes formed is lower than the amount found in a sample taken from a healthy subject, the subject from whom the sample examined has been taken is likely to suffer from a disease.

Any data demonstrating the presence or absence of the complex comprising the antibody and the inventive polypeptide may be correlated with reference data. For example, detection of said complex indicates that the patient who provided the sample analyzed has suffered, is suffering or is likely to suffer in the future from a disease. If a patient has been previously diagnosed and the method for obtaining diagnostically relevant information is run again, the amount of complex detected in both runs may be correlated to find out about the progression of the disease and/or the success of a treatment. In a preferred embodiment, any information or data demonstrating the presence of absence of the complex may be communicated to the patient or a medical doctor treating the patient, preferably by telephone, in a written form or via the internet, for example as an email or text message.

In another preferred embodiment, the prognosis, diagnosis, methods or test kit in line with the inventive teachings contemplate the use of indirect immunofluorescence. The person skilled in the art is familiar with such techniques and the preparation of suitable samples, which are described in the state of the art (US4647543; Voigt, J., Krause, C., Rohwäder, E, Saschenbrecker, S., Hahn, M., Danckwardt, M., Feirer, C., Ens, K, Fechner, K, Barth, E, Martinetz, T., and Stöcker, W. (2012), Automated Indirect Immunofluorescence Evaluation of Antinuclear Autoantibodies on HEp-2 Cells," Clinical and Developmental Immunology, vol. 2012, doi:10.1155/2012/65105; Bonilla, E., Francis, L., Allam, F., et al., Immuno-fluorescence microscopy is superior to fluorescent beads for detection of antinuclear antibody reactivity in systemic lupus erythematosus patients, Clinical Immunology, vol. 124, no. 1, pp. 18-21, 2007). Suitable reagents, devices and software packages are commercially available, for example from EUROIMMUN, Lübeck, Germany.

A sample may be subjected to a test to determine only whether an autoantibody binding to DAGLA is present, but it is preferred that diagnostic methods, tests, devices and the like contemplate determining the presence of autoantibodies to one or more additional polypeptides, preferably related to neurological autoimmune diseases, preferably selected from, more preferably all from the group comprising Hu, Yo, Ri, CV2, PNMA1, PNMA2, DNER/Tr, ARHGAP26, ITPR1, ATP1A3, NBC1, Neurochrondrin, CARPVIII, Zic4, Sox1, Ma, MAG, MP0, MBP, GAD65, amphiphysin, recoverin, GABA A receptor (EP13189172.3), GABA B receptor (EP2483417), glycine receptor, gephyrin, IgLON5 (US2016/0349275), DPPX (US2015/0247847), aquaporin-4, MOG, NMDA receptor, AMPA receptors, GRM1, GRM5, LGI1, VGCC und mGluR1 and CASPR2, which antigens are preferably immobilized, for example on a medical device such as a line blot. The diagnostically relevant markers Neurochrondrin (EP15001186), ITPR1 (EP14003703.7), NBC1 (EP14003958.7), ATP1A3, also referred to as alpha 3 subunit of human neuronal Na(+)/K(+) ATPase (EP14171561.5), Flotillin1/2 (EP3101424), NSF, STX1B and VAMP2 (EP17001205.8) and RGS8 (EP17000666.2), autoantibodies to one or more of which, preferably all, may be detected in addition, have been described in the state of the art.

According to the teachings of the present invention, an antibody, preferably an autoantibody binding to the inventive polypeptide used for the diagnosis of a disease is provided. The person skilled in the art is familiar with methods for purifying antibodies, for example those described in Hermanson, G. T., Mallia, A. K., and Smith, P. K. (1992), Immobilized Affinity Ligand Techniques, San Diego: Academic Press. Briefly, an antigen binding specifically to the antibody of interest, which antigen is the inventive polypeptide, is immobilized and used to purify, via affinity chromatography, the antibody of interest from an adequate source. A liquid sample comprising antibodies from a patient suffering from the neurological disorder identified by the inventors may be used as the source.

According to the invention, an antibody, which is an autoantibody, is provided that is capable of binding specifically to DAGLA. Vice versa, a variant of DAGLA binds specifically to an autoantibody binding specifically to DAGLA. In a preferred embodiment, the term "antibody", as used herein, refers to any immunoglobulin-based binding moieties, more preferably one comprising at least one immunoglobulin heavy chain and one immunoglobulin light chain, including, but not limited to monoclonal and polyclonal antibodies as well as variants of an antibody, in particular fragments, which binding moieties are capable of binding to the respective antigen, more preferably binding specifically to it. In a preferred embodiment, the term "binding specifically", as used herein, means that the binding is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7. The antibody may be part of an autoantibody preparation which is heterogeneous or may be a homogenous autoantibody, wherein a heterogeneous preparation comprises a plurality of different autoantibody species as obtainable by preparation from the sera of human donors, for example by affinity chromatography using the immobilized antigen to purify any autoantibody capable of binding to said antigen. The antibody may be glycosylated or non-glycosylated. The antibody may be a recombinant and/or monoclonal mammalian antibody, preferably an animal which is not a human. The antibody may be an antibody binding specifically against SEQ ID NO 26 or SEQ ID NO 28, preferably SEQ ID NO28.

The person skilled in the art is familiar with methods that may be used for the identification, production and purification of antibodies and variants thereof, for examples those described in EP 2 423 226 A2 and references therein. Preferably the antibody is purified and/or recombinant. Preferably the antibody is bound to the diagnostically useful carrier.

The present invention provides a method for isolating an autoantibody binding to DAGLA, comprising the steps a) contacting a sample comprising the antibody with the inventive polypeptide such that a complex is formed, b) isolating the complex formed in step a), c) dissociating the complex isolated in step b), and d) separating the antibody from the inventive polypeptide. A sample from a patient suffering from the novel neurological disorder identified by the inventors may be used as the source of antibody. Suitable methods are described in the state of the art, for example in the Handbooks "Affinity chromatography", "Strategies for Protein Purification" and "Antibody Purification" (2009/2010), published by GE Healthcare Life Sciences, and in in Philips, Terry, M., Analytical techniques in immunochemistry, 1992, Marcel Dekker, Inc.

The present teaching provides a pharmaceutical composition comprising the inventive polypeptide, which composition is preferably suitable for administration to a subject, preferably a mammalian subject, more preferably to a human. Such a pharmaceutical composition may comprise a pharmaceutically acceptable carrier. The pharmaceutical composition may, for example, be administered orally, parenterally, by inhalation spray, topically, by eyedrops, rectally, nasally, buccally, vaginally or via an implanted reservoir, wherein the term "parentally", as used herein, comprises subcutaneous, intracutaneous, intravenous, intramuscular, intra-articular, intrasynovial, intrasternal, intrathecal, intralesional and intracranial injection or infusion techniques. The pharmaceutical composition may be provided in suitable dosage forms, for example capsules, tablets and aqueous suspensions and solutions, preferably in sterile form. It may be used in a method of treatment of a disease, which method comprises administering an effective amount of the inventive polypeptide to a subject. In a preferred embodiment, the present teaching provides a vaccine comprising the inventive polypeptide, optionally comprising an auxiliary agent such as an adjuvans or a buffer, and the use of the inventive polypeptide for the preparation of a vaccine.

Within the scope of the present invention, a medical or diagnostic device comprising, preferably coated with a reagent for detecting the inventive (auto)antibody and/or the inventive polypeptide is provided. Preferably such a medical or diagnostic device comprises the inventive polypeptide in a form that allows contacting it with an aqueous solution, more preferably the liquid human sample, in a straightforward manner. In particular, the inventive polypeptide comprising may be immobilized on the surface of a carrier, preferably selected from the group comprising glass plates or slides, biochips, microtiter plates, beads, for example magnetic beads, apharesis devices, chromatography columns, membranes or the like. Exemplary medical devices include line blots, microtiter plates, glass slides for microscopy, beads, preferably magnetic beads, and biochips. In addition to the inventive polypeptide, the medical or diagnostic device may comprise additional polypeptides, for example positive or negative controls such as samples comprising or not comprising an antibody binding to the polypeptide of interest, or known other antigens binding to autoantibodies of diagnostic value, particularly those related other diseases associated with one or more identical or similar symptoms.

The inventive teachings provide a kit, preferably for diagnosing a disease. Such a kit may comprise instructions detailing how to use the kit and a means for contacting the inventive polypeptide with a bodily fluid sample from a subject, preferably a human subject, for example a line blot, wherein the inventive polypeptide is immobilized on the line blot. Furthermore, the kit may comprise a positive control, for example a batch of autoantibody or recombinant antibody known to bind to the polypeptide according to the present invention, preferably against SEQ ID NO 26 and/or SEQ ID NO28, and a negative control, for example a protein having no detectable affinity to the inventive polypeptide such as bovine serum albumin. Finally, such a kit may comprise one or more standard solutions, also referred to as calibrator, of an antibody binding to DAGLA, preferably SEQ ID NO 26 and/or SEQ ID NO28, preferably with a known absolute or relative concentration, for preparing a calibration curve. Preferably the kit comprises two calibrators, wherein the first calibrator has a concentration of the antibody that is no more than 50, 40, 30, 20, 10 5, 2.5 or 1 % of the concentration of the antibody in the second calibrator. In a preferred embodiment, a device comprising the diagnostically useful carrier is calibrated by detecting the antibody concentration in at least two calibrators and obtaining a concentration value for each calibrators, preferably three, four or five or more calibrators, followed by setting up a standard calibration curve.

In a preferred embodiment, the kit comprises a means for detecting an autoantibody binding to the inventive polypeptide, preferably by detecting a complex comprising the inventive polypeptide and an antibody binding to the inventive polypeptide. Such means is preferably an agent that binds to said complex and modifies the complex or carries a label such that makes the complex detectable. For example, said means may be a labeled antibody binding to said polypeptide, at a binding site other than the binding site recognized by the primary antibody or to a constant region of the primary antibody. Alternatively, said means may be a secondary antibody binding to the constant region of the autoantibody, preferably a secondary antibody specific for mammalian IgG class of antibodies. A multitude of methods and means for detecting such a complex have been described in the state of the art, for example in Philips, Terry, M., Analytical techniques in immunochemistry, 1992, Marcel Dekker, Inc.

DAGLA or a variant thereof may be produced or provided in the form of a cell comprising and/or expressing a nucleic acid encoding said polypeptide. If a nucleic acid comprising a sequence that encodes for the inventive polypeptide or variant thereof is used, such a nucleic acid may be an unmodified nucleic acid. In a preferred embodiment, the nucleic acid is a nucleic acid that, as such, does not occur in nature and comprises, compared to natural nucleic acid, at least one modification, for example an isotopic content or chemical modifications, for example a methylation, sequence modification, label or the like indicative of synthetic origin. In a preferred embodiment, the nucleic acid is a recombinant nucleic acid or part or a nucleic acid, and is, in a more preferred embodiment, part of a vector, in which it may be functionally linked with a promoter that allows for expression, preferably overexpression of the nucleic acid. The person skilled in the art is familiar with a variety of suitable vectors, of which are commercially available, for example from Origene. For example, a vector encoding for fusion constructs with a C-terminal GFP may be used. The cell may be a eukaryotic or prokaryotic cell, preferably of eukaryotic cell, such as a yeast cell, and is more preferably a mammalian, more preferably a human cell such as a HEK293 cell. Examples of a mammalian cell include a HEK293, CHO or COS-7 cell. The cell comprising the nucleic acid encoding for the inventive polypeptide may be a recombinant cell or an isolated cell wherein the term "isolated" means that the cell is enriched such that, compared to the environment of the wild type of said cell, fewer cells of other differentiation or species or in fact no such other cells are present. In a preferred embodiment, the vector encodes for a polypeptide comprising SEQ ID NO 26 and/or SEQ ID NO28, preferably SEQ ID NO28.

In a preferred embodiment, the medical device according to the present invention, preferably a slide suitable for microscopy, comprises one or more, preferably all reagents from the group comprising a first eukaryotic cell expressing, preferably overexpressing DAGLA or a variant thereof, a eukaryotic, preferably mammalian tissue expressing endogenous DAGLA such as rat or primate cerebellum, a second eukaryotic cell, which is the same type of cell as the first eukaryotic cell, but does not express or overexpress DAGLA. The first and the second eukaryotic cell are cultured cells derived from an isolated cell line such as HEK293. Preferably, the first and the second cell are each transfected with a vector sharing the same backbone, wherein the vector used to transfect the first cell comprises a nucleic acid encoding DAGLA or a variant thereof and the vector used to transfect the second cell does not comprise DAGLA or a variant thereof. The second cell may serve as a negative control. The reagents may be spatially separate on the medical device, such that they may be evaluated independently, with no antigen from one reagent contaminating another. In a more preferred embodiment, the first and/or the second cell is a fixed cell, for example fixed using methanol or acetone. Protocols for fixing cells are described in the state of the art. As an additional reagent, a secondary labeled antibody, preferably labeled with a fluorescent dye may be provided. The reagents and the medical device may be part of a kit.

In a preferred embodiment, a microtiter plate, membrane, blot such as dot blot or line blot is used to carry out the diagnostic method according to the invention. The person skilled in the art is familiar with the experimental setup of a line blot, which is described in the state of the art (Raoult, D., and Dasch, G. A. (1989), The line blot: an immunoassay for monoclonal and other antibodies. Its application to the serotyping of gram-negative bacteria. J. Immunol. Methods, 125 (1- 2), 57-65; WO2013041540). If the medical device is a line blot, it may comprise DAGLA or a variant thereof immobilized on a membrane, preferably in the shape of a test stripe. The membrane may comprise one or more additional antigens, spatially separated from DAGLA. The membrane may comprise a control band indicating addition of the sample such as a blood sample and/or a control band indicating addition of a secondary antibody. A kit may comprise any component, preferably all from the group comprising the line blot, a secondary antibody and a washing solution.

In another preferred embodiment, the medical device is a microtiter plate comprising at least 8 wells. At least one of the wells is directly or indirectly coated with DAGLA or a variant thereof. At least 3, preferably 4, more preferably 5 calibrators are provided that comprise an antibody to DAGLA at a defined concentration and may be used to set up a calibration curve for semi-quantitative analysis. A secondary antibody comprising an enzymatically active label may be provided. A kit may comprise any component, preferably all from the group comprising the microtiter plate, the calibrators, a washing solution and the secondary antibody.

In another preferred embodiment, the medical device is a bead coated directly or indirectly with DAGLA or a variant thereof. The bead may be selected from the group comprising a magnetic bead and a fluorescent bead. A secondary antibody comprising a label capable or chemiluminescence or fluorescence may be provided. A positive control comprising an antibody to DAGLA may be provided. At least 3, preferably 4, more preferably 5 calibrators may be provided that comprise an antibody to DAGLA at a defined concentration and may be used to set up a calibration curve for semi-quantitative analysis. If the label is capable of generating chemiluminescence, a solution may be provided that comprises additional components required for the chemiluminescence reaction. For example, if the label is an enzyme, the solution comprises substrates. If the label is a compound capable of generating chemiluminescence such as an acridinium ester, additional compounds required for the reaction are provided in the solution. A kit may comprise any component, preferably all from the group comprising the bead, the secondary antibody, the calibrators, a washing solution and the solution comprising additional components.

In a preferred embodiment, the present teaching provides a use of a reagent for the detection of an autoantibody to DAGLA or a reagent binding to such autoantibody, or of a nucleic acid encoding DAGLA or the variant or a nucleic acid hybridizing specifically to a nucleic acid encoding DAGLA or a vector or cell comprising said nucleic acid for the manufacture of kit for the diagnosis of a disease.

In a preferred embodiment, any method or use according to the present invention may be intended for a non-diagnostic use, i.e. determining the presence of an autoantibody to binding to DAGLA for a use other than diagnosing a patient. For example, the method or use may be for testing *in vitro* the efficiency of a medical device designed to remove an autoantibody from a patient's blood, wherein the testing is performed on a liquid other than patient's blood. After the use of the medical device with a patient, its capacity to remove autoantibody may be checked by running a solution comprising antibody to DAGLA through the device, followed by use of the method according to the present invention to confirm that less or no antibody is in the solution that has been passed through the device, i.e. showing that the device has still the capacity to remove antibody from the solution.

In another preferred embodiment, the method may be for confirming the reliability of a diagnostic assay and may involve detecting an antibody to DAGLA in a solution, which is not a sample from a patient, but is known to comprise an antibody to DAGLA, preferably at a known concentration. Alternatively, the solution may be a negative control not comprising the antibody to check the background. Such method may be run in parallel with, after or before a diagnostic method. In a preferred embodiment, any method or use according to the present invention may be intended for generating an autoantibody profile, preferably for detecting a disease in a mammal, preferably a human. In a preferred embodiment, any method or use may be for detecting disease-associated markers in a sample from neurological disease patients.

In a preferred embodiment, any method or use according to the present invention may be for identifying a subject at risk of suffering from or developing a neurological disease and/or a tumor.

In a preferred embodiment, the present teaching provides an apparatus for analyzing a sample from a patient to detect an autoantibody against DAGLA, preferably SEQ ID NO26 and/or SEQ ID NO28, indicating an increased likelihood of a disease or of developing a disease, comprising:
a. a carrier, which contains a means for capturing the autoantibody from the sample when the sample is contacted with the carrier,
b. a detectable means capable of binding to the antibody captured by the carrier when the detectable means is contacted with the carrier, wherein the detectable means is a labeled secondary antibody capable of binding to the antibody captured on the carrier,
c. optionally a means for removing any sample from the carrier and the detectable means, preferably by washing;
d. a detecting device for detecting the presence of the detectable means and converting the results into an electrical signal, and

optionally a means for receiving the electronical signal from the detecting device and determining if the level of the signal is indicative of an increased likelihood of having or developing a disease, by comparing with the level of signal detected in the background or an input reference value obtained with samples from healthy subjects or by comparing the level of signal obtained with one sample with the level of signal obtained with a second sample obtained at a later time point, preferably at least one month later.

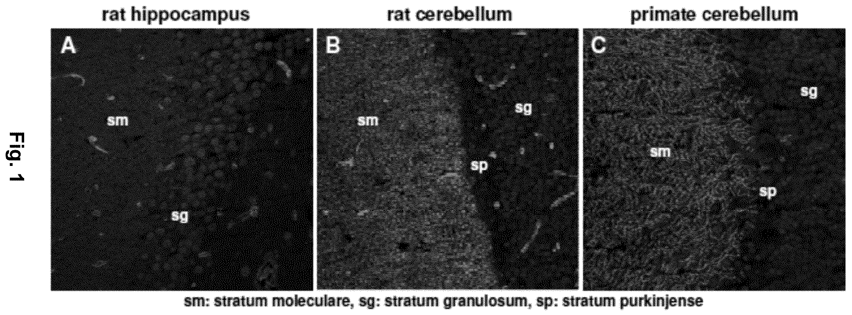

Fig. 1 shows the results of indirect immunofluorescence assays of sera from patients P1 to P5 using permeabilized cryosections of rat and primate cerebellum. Granular staining of the molecular layer was observed.

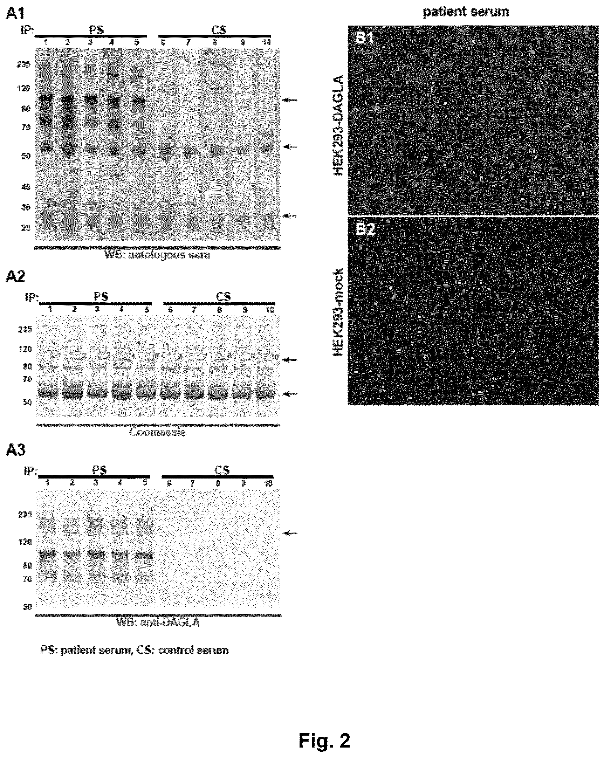

Fig. 2 shows immunoprecipitation of DAGLA from homogenized rat cerebellum using patient sera followed by Western Blot analysis using patient sera as antibody source (A1), Coomassie-stained SDS PAGE (A2), Western Blot using a commercial recombinant antibody binding to DAGLA (A3). Furthermore, the patients' samples were tested by indirect immunofluorescence using transfected HEK293 cells expressing DAGLA. Patients' sera reacted with the DAGLA-expressing cells (B1). In contrast, mock-transfected cell did not demonstrate any specific antibody binding (B2).

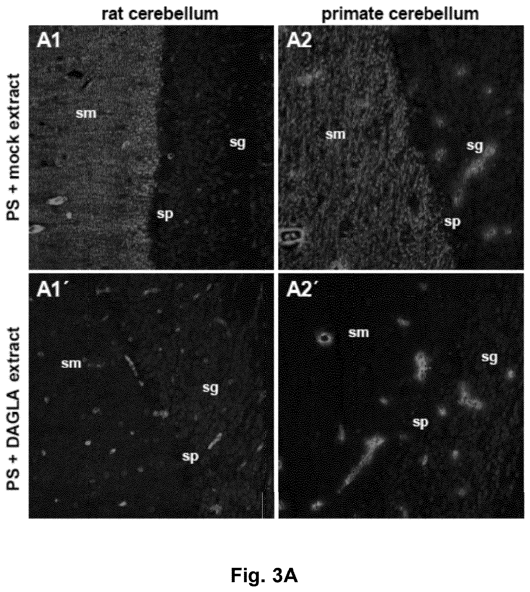

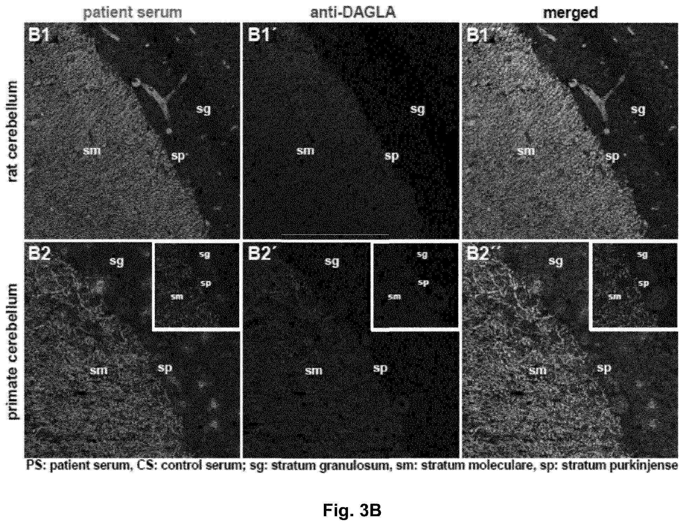

Fig. 3 shows the results of immunofluorescence on rat and primate cerebellum using autoantibodies from patients that were or were not pre-incubated with DAGLA. The reaction of the patients' auto-antibodies on tissue could be abolished by pre-incubation with HEK293 lysate containing DAGLA (SEQ ID NO 4) (Fig. 3A). Antibody binding was unaffected when a comparable fraction from mock-transfected HEK293 cells was used.

Indirect immunofluorescence with rat and primate cerebellum tissue using patient serum and a specific polyclonal anti-DAGLA antibody (Sigma-Aldrich, Germany) revealed an exact overlap of the molecular layer staining (Fig. 3B).

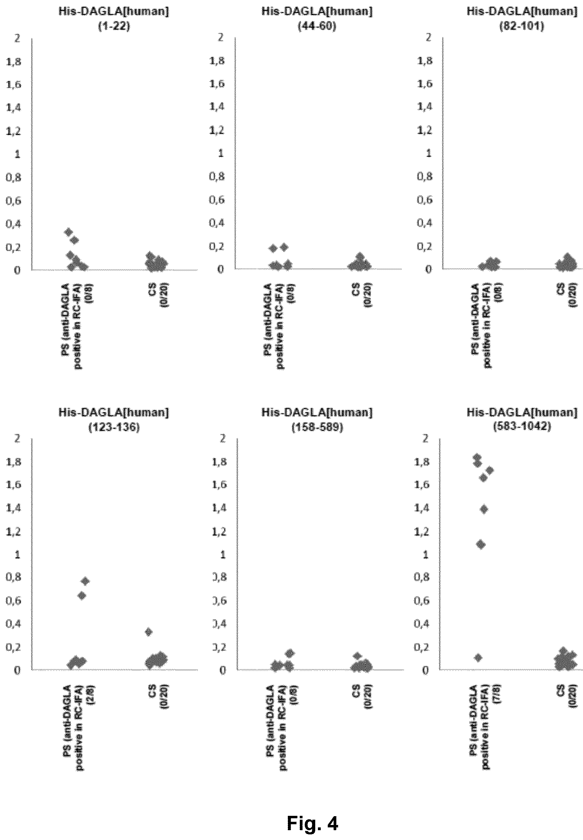

**Fig.** 4 shows an ELISA analysis of eight positive patient sera comprising autoantibodies to DAGLA using six different fragments of human DAGLA. Two fragments (583-1042 and 123-136) yielded positive results.

The present application comprises a range of sequences, more specifically:
**SEQ ID NO 1 (cDNA encoding human DAGLA)** 
atgcccgggatcgtggtgttccggcggcgctggtctgtgggcagtgatgacctcgtcctaccagccatcttcctctttctcctgcataccac
ctggtttgtgatcctgtccgtggtgctcttcggcctggtctataacccgcacgaggcctgctccctgaacctggtggaccacggccgcgg
ctacctgggcatcctgctgagctgcatgatcgctgagatggccatcatctggctgagcatgcgcgggggcatcctctacacggagccc
cgtgactccatgcagtacgtgctctacgtgcgcctggccatcctggtgatcgagttcatctacgccatcgtgggcatcgtctggctcactc
agtactacacctcctgcaacgacctcactgccaagaatgtcaccctcggaatggttgtctgcaactgggtagtcatcctcagtgtgtgca
tcactgtcctctgcgtcttcgaccccacgggccgcacctttgtcaagctgagagccaccaagaggaggcagcgtaacctgcggacct
acaacctgcggcaccgcttagaggagggtcaagccaccagctggtcgcgccggctcaaagtgttcctctgctgcacgcggacgaa
ggactcccagtcagatgcctactcagaaatcgcctacctctttgcggagttcttccgggaccttgacattgtgccatccgacatcattgct
ggcctggtgctgctccggcagcggcagcgggccaagcgcaacgccgtgctggacgaggcaaacaatgacatcttggccttcctgtc
tgggatgccggtgaccagaaacaccaagtacctcgacctcaagaattcacaagagatgctccgctacaaagaggtctgctactaca
tgctctttgccctggctgcctacgggtggcccatgtacctgatgcggaagcccgcctgcggcctctgccaactggctcggtcctgctcgt
gttgcctgtgtcctgcgaggccgcggttcgcccctggagtcaccatcgaggaagacaactgctgtggctgtaatgccattgccatccgg
cgccacttcctggacgagaacatgactgcggtggacatcgtctatacctcctgccatgatgcggtctatgaaacgcccttctacgtggc
ggtggaccatgacaagaagaaagtggtgatcagtatccgggggaccctgtcccccaaggatgccctgactgacctgacgggtgatg
ctgagcgcctccccgtggaggggcaccacggcacctggctgggccacaagggtatggtcctctcagctgagtacatcaagaagaa
actggagcaggagatggtcctgtcccaggcctttgggcgagacctgggccgcggaaccaaacactacggcctgattgtggtgggcc
actccctgggcgcgggcactgctgccatcctctccttccttctgcgcccacagtatccgaccctcaagtgctttgcctactccccgccagg
gggcctgctgagtgaggatgcaatggagtattccaaggagttcgtgactgctgtggttctgggcaaagacctcgtccccaggattggcc
tctctcagctggaaggcttccgcagacagctcctggatgtcctgcagcgaagcaccaagcccaaatggcggatcatcgtgggggcc
accaaatgcatccccaagtcggagctgcctgaggaggtagaggtgaccaccctggccagcacgcggctctggacccaccccagc
gacctaactatagccctctcagccagcactccactctacccgcccggccgcatcatccacgtggtccacaaccaccctgcagagca
gtgctgctgctgtgagcaggaggagcccacatactttgccatctggggcgacaacaaggccttcaatgaggtgatcatctcgccagcc
 
atgctgcatgagcacctgccctatgtggtcatggaggggctcaacaaggtgctggagaactacaacaaggggaagaccgctctgct
ctctgcagccaaggtcatggtgagccctaccgaggtggacctgactcctgagctcatcttccagcagcagccactccccacggggcc
gcccatgcccactggccttgccctggagctgccgactgcagaccaccgcaacagcagcgtcaggagcaagtcccagtctgagatg
agcctggagggcttctcggaggggcggctgctgtcgccagtggttgcggcggcccgccaggacccggtggagctgctgctgct
gtctacccaggagcggctggcggcggagctgcaggcccggcgggcaccactggccaccatggagagcctctcggacactgagtc
cctgtacagcttcgactcgcgccgctcctcaggcttccgcagcatccggggctcccccagcctccacgctgtgctggagcgtgatgaa
ggccacctcttctacattgaccctgccatccccgaggaaaacccatccctgagctcgcgcactgagctgctggcggccgacagcctgt
ccaagcactcacaggacacgcagcccctggaggcggccctgggcagtggcggcgtcactcctgagcggcccccagtgctgcgg
ccaatgacgaggaggaagaggttggcggtgggggtggcgggccggcctcccgcggggagctggcgctgcacaatgggcgcctg
ggggactcgcccagtcctcaggtgctggaattcgccgagttcatcgacagcctcttcaacctggacagcaagagcagctccttccaa
gacctctactgcatggtggtgcccgagagccccaccagtgactacgctgagggccccaagtcccccagccagcaagagatcctgct
ccgtgcccagttcgagcccaacctggtgcccaagcccccacggctctttgccggctcagccgacccctcctcgggcatctcactctcg
ccctccttcccgctcagctcctcgggtgagctcatggacctgacgcccacgggcctcagtagccaggaatgcctggcggctgacaag
atccggacttctacccccactggccacggagccagccccgccaagcaagatgagctggtcatctcagcacgctag
SEQ ID NO 2 [sense DAGLA primer for amplifying cDNA]
   ATACGTCTCACATGCCCGGGATCGTGGTGTTCCGG
SEQ ID NO 3 [asense DAGLA-Stop primer for amplifying cDNA]
   ATACGTCTCCTCGAGCTAGCGTGCTGAGATGACCAGCTC
SEQ ID NO 4 (DAGLA as expressed in HEK293) 
MPGIVVFRRRWSVGSDDLVLPAIFLFLLHTTWFVILSVVLFGLVYNPHEACSLNLVDHGRGYLGI
LLSCMIAEMAIIWLSMRGGILYTEPRDSMQYVLYVRLAILVIEFIYAIVGIVWLTQYYTSCNDLTAK
NVTLGMVVCNWVVILSVCITVLCVFDPTGRTFVKLRATKRRQRNLRTYNLRHRLEEGQATSWS
RRLKVFLCCTRTKDSQSDAYSEIAYLFAEFFRDLDIVPSDIIAGLVLLRQRQRAKRNAVLDEANN
DILAFLSGMPVTRNTKYLDLKNSQEMLRYKEVCYYMLFALAAYGWPMYLMRKPACGLCQLAR
SCSCCLCPARPRFAPGVTIEEDNCCGCNAIAIRRHFLDENMTAVDIVYTSCHDAVYETPFYVAV
DHDKKKVVISIRGTLSPKDALTDLTGDAERLPVEGHHGTWLGHKGMVLSAEYIKKKLEQEMVLS
QAFGRDLGRGTKHYGLIVVGHSLGAGTAAILSFLLRPQYPTLKCFAYSPPGGLLSEDAMEYSKE
FVTAVVLGKDLVPRIGLSQLEGFRRQLLDVLQRSTKPKWRIIVGATKCIPKSELPEEVEVTTLAST
RLWTHPSDLTIALSASTPLYPPGRIIHVVHNHPAEQCCCCEQEEPTYFAIWGDNKAFNEVIISPA
MLHEHLPYVVMEGLNKVLENYNKGKTALLSAAKVMVSPTEVDLTPELIFQQQPLPTGPPMPTG
 
LALELPTADHRNSSVRSKSQSEMSLEGFSEGRLLSPVVAAAARQDPVELLLLSTQERLAAELQA
RRAPLATMESLSDTESLYSFDSRRSSGFRSIRGSPSLHAVLERDEGHLFYIDPAIPEENPSLSSR
TELLAADSLSKHSQDTQPLEAALGSGGVTPERPPSAAANDEEEEVGGGGGGPASRGELALHN
GRLGDSPSPQVLEFAEFIDSLFNLDSKSSSFQDLYCMVVPESPTSDYAEGPKSPSQQEILLRAQ
FEPNLVPKPPRLFAGSADPSSGISLSPSFPLSSSGELMDLTPTGLSSQECLAADKIRTSTPTGH
GASPAKQDELVISAR
**SEQ ID NO 5 [sense DAGLA[1-22] primer for cloning DAGLA fragment]** 
CATGCCCGGGATCGTGGTGTTCCGGCGGCGCTGGTCTGTGGGCAGTGATGACCTCGTCC
TACCAGCCTAATGA
**SEQ ID NO 6 [asense DAGLA[1-22] primer for cloning DAGLA fragment]** 
TCGATCATTAGGCTGGTAGGACGAGGTCATCACTGCCCACAGACCAGCGCCGCCGGAACA
CCACGATCCCGGG
**SEQ ID NO 7 [sense DAGLA[44-60] primer for cloning DAGLA fragment]** 
CATGGTCTATAACCCGCACGAGGCCTGCTCCCTGAACCTGGTGGACCACGGCCGCTAATG
A
**SEQ ID NO 8 [asense DAGLA[44-60] primer for cloning DAGLA fragment]** 
TCGATCATTAGCGGCCGTGGTCCACCAGGTTCAGGGAGCAGGCCTCGTGCGGGTTATAGA
C
**SEQ ID NO 9 [sense DAGLA[81-101] primer for cloning DAGLA fragment]** 
CATGCGCGGGGGCATCCTCTACACGGAGCCCCGTGACTCCATGCAGTACGTGCTCTACGT
GCGCTAATGA
**SEQ ID NO 10 [asense DAGLA[81-101] primer for cloning DAGLA fragment]** 
TCGATCATTAGCGCACGTAGAGCACGTACTGCATGGAGTCACGGGGCTCCGTGTAGAGGA
TGCCCCCGCG
**SEQ ID NO 11 [sense DAGLA[123-136] primer for cloning DAGLA fragment]**
   CATGTACACCTCCTGCAACGACCTCACTGCCAAGAATGTCACCCTCTAATGA
**SEQ ID NO 12 [asense DAGLA[123-136] primer for cloning DAGLA fragment]**
   TCGATCATTAGAGGGTGACATTCTTGGCAGTGAGGTCGTTGCAGGAGGTGTA
**SEQ ID NO 13 (H8-GST-DAGLA[1-22])** 
MSHHHHHHHHMSPILGYWKIKGLVQPTRLLLEYLEEKYEEHLYERDEGDKWRNKKFELGLEFP
NLPYYIDGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKLVCFKKR
IEAIPQIDKYLKSSKYIAWPLQGWQATFGGGDHPPKLEVLFQGPAMPGIVVFRRRWSVGSDDL
VLPA
**SEQ ID NO 14 (H8-GST-DAGLA[44-60])** 
MSHHHHHHHHMSPILGYWKIKGLVQPTRLLLEYLEEKYEEHLYERDEGDKWRNKKFELGLEFP
NLPYYIDGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKLVCFKKR
IEAIPQIDKYLKSSKYIAWPLQGWQATFGGGDHPPKLEVLFQGPAMVYNPHEACSLNLVDHGR
**SEQ ID NO 15 (H8-GST-DAGLA[81-101])** 
MSHHHHHHHHMSPILGYWKIKGLVQPTRLLLEYLEEKYEEHLYERDEGDKWRNKKFELGLEFP
NLPYYIDGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKLVCFKKR
IEAIPQIDKYLKSSKYIAWPLQGWQATFGGGDHPPKLEVLFQGPAMRGGILYTEPRDSMQYVLY
VR
**SEQ ID NO 16 (H8-GST-DAGLA[123-136])** 
MSHHHHHHHMSPILGYWKIKGLVQPTRLLLEYLEEKYEEHLYERDEGDKWRNKKFELGLEFP
NLPYYIDGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKLVCFKKR
IEAIPQIDKYLKSSKYIAWPLQGWQATFGGGDHPPKLEVLFQGPAMYTSCNDLTAKNVTL
SEQ ID NO 17 [sense DAGLA[158-598]]
   ATACGTCTCGCATGGACCCCACGGGCCGCACCTTTGTCAAG
**SEQ ID NO 18 [asense DAGLA[158-598]]**
   TATCGTCTCGTCGATCATTATGGAGTGCTGGCTGAGAGGGCTATAG
**SEQ ID NO 19 [sense DAGLA[583-1042]]**
   ATACGTCTCGCATGTGGACCCACCCCAGCGACCTAACTATAGC
**SEQ ID NO 20 [asense DAGLA[583-1042]]**
   TATCGTCTCGTCGATCATTAGCGTGCTGAGATGACCAGCTCATCTTG
**SEQ ID NO 21 (H8-DAGLA[158-598])** 
MSHHHHHHHHSMDPTGRTFVKLRATKRRQRNLRTYNLRHRLEEGQATSWSRRLKVFLCCTR
TKDSQSDAYSEIAYLFAEFFRDLDIVPSDIIAGLVLLRQRQRAKRNAVLDEANNDILAFLSGMPVT
RNTKYLDLKNSQEMLRYKEVCYYMLFALAAYGWPMYLMRKPACGLCQLARSCSCCLCPARPR
FAPGVTIEEDNCCGCNAIAIRRHFLDENMTAVDIVYTSCHDAVYETPFYVAVDHDKKKVVISIRG
TLSPKDALTDLTGDAERLPVEGHHGTWLGHKGMVLSAEYIKKKLEQEMVLSQAFGRDLGRGT
KHYGLIVVGHSLGAGTAAILSFLLRPQYPTLKCFAYSPPGGLLSEDAMEYSKEFVTAVVLGKDLV
PRIGLSQLEGFRRQLLDVLQRSTKPKWRIIVGATKCIPKSELPEEVEVTTLASTRLWTHPSDLTIA
LSASTP
**SEQ ID NO 22 (H8-DAGLA[583-1042])** 
MSHHHHHHHHSMWTHPSDLTIALSASTPLYPPGRIIHVVHNHPAEQCCCCEQEEPTYFAIWGD
NKAFNEVIISPAMLHEHLPYVVMEGLNKVLENYNKGKTALLSAAKVMVSPTEVDLTPELIFQQQP
LPTGPPMPTGLALELPTADHRNSSVRSKSQSEMSLEGFSEGRLLSPVVAAAARQDPVELLLLS
TQERLAAELQARRAPLATMESLSDTESLYSFDSRRSSGFRSIRGSPSLHAVLERDEGHLFYIDP
AIPEENPSLSSRTELLAADSLSKHSQDTQPLEAALGSGGVTPERPPSAAANDEEEEVGGGGGG
PASRGELALHNGRLGDSPSPQVLEFAEFIDSLFNLDSKSSSFQDLYCMVVPESPTSDYAEGPK
SPSQQEILLRAQFEPNLVPKPPRLFAGSADPSSGISLSPSFPLSSSGELMDLTPTGLSSQECLA
ADKIRTSTPTGHGASPAKQDELVISAR
**SEQ ID NO 23 (H8-GST-DAGLA[1-22])**
   MPGIVVFRRRWSVGSDDLVLPA
**SEQ ID NO 24 (H8-GST-DAGLA[44-60])**
   VYNPHEACSLNLVDHGR
**SEQ ID NO 25 (H8-GST-DAGLA[81-101])**
   MRGGILYTEPRDSMQYVLYVR
**SEQ ID NO 26 (H8-GST-DAGLA[123-136]), reactive with patient sera**
   YTSCNDLTAKNVTL
**SEQ ID NO 27 (DAGLA[158-598])** 
TGRTFVKLRATKRRQRNLRTYNLRHRLEEGQATSWSRRLKVFLCCTRTKDSQSDAYSEIAYLF
AEFFRDLDIVPSDIIAGLVLLRQRQRAKRNAVLDEANNDILAFLSGMPVTRNTKYLDLKNSQEML
RYKEVCYYMLFALAAYGWPMYLMRKPACGLCQLARSCSCCLCPARPRFAPGVTIEEDNCCGC
NAIAIRRHFLDENMTAVDIVYTSCHDAVYETPFYVAVDHDKKKVVISIRGTLSPKDALTDLTGDAE
RLPVEGHHGTWLGHKGMVLSAEYIKKKLEQEMVLSQAFGRDLGRGTKHYGLIVVGHSLGAGT
AAILSFLLRPQYPTLKCFAYSPPGGLLSEDAMEYSKEFVTAVVLGKDLVPRIGLSQLEGFRRQLL
DVLQRSTKPKWRIIVGATKCIPKSELPEEVEVTTLASTRLWTHPSDLTIALSASTPPD
**SEQ ID NO 28 (DAGLA[583-1042]), reactive with patient sera**

WTHPSDLTIALSASTPLYPPGRIIHVVHNHPAEQCCCCEQEEPTYFAIWGDNKAFNEVIISPAML
HEHLPYVVMEGLNKVLENYNKGKTALLSAAKVMVSPTEVDLTPELIFQQQPLPTGPPMPTGLAL
ELPTADHRNSSVRSKSQSEMSLEGFSEGRLLSPVVAAAARQDPVELLLLSTQERLAAELQARR
APLATMESLSDTESLYSFDSRRSSGFRSIRGSPSLHAVLERDEGHLFYIDPAIPEENPSLSSRTE
LLAADSLSKHSQDTQPLEAALGSGGVTPERPPSAAANDEEEEVGGGGGGPASRGELALHNGR
LGDSPSPQVLEFAEFIDSLFNLDSKSSSFQDLYCMVVPESPTSDYAEGPKSPSQQEILLRAQFE
PNLVPKPPRLFAGSADPSSGISLSPSFPLSSSGELMDLTPTGLSSQECLAADKIRTSTPTGHGA
SPAKQDELVISAR

The present invention is further illustrated by the following non-limiting examples from which further features, embodiments, aspects and advantages may be taken.

### Examples

### Summary

Methods: Five patients (P1 - P5) suffering from neurological conditions underwent serological investigation. For this purpose, sera from all five patients were subjected to comprehensive autoantibody screening by indirect immunofluorescence assay (IFA) and immunoblot. Immunoprecipitation with lysates of cerebellum followed by mass spectrometry (MS) was used to identify the autoantigen, which was verified by Western blot (WB) with monospecific animal antibody against the respective target antigen as well as by recombinant expression in HEK293 cells and use of the recombinant protein in immunoassays. Furthermore, sera of patients with neurological symptoms and defined anti-neural autoantibodies, sera with a similar staining pattern as P1 to P5 without known autoantibody reactivity, as well as negative control sera were screened for anti-DAGLA antibodies. Six different fragments of the DAGLA protein were recombinantly expressed in *E*. *coli.* Purified proteins were analyzed in ELISA using anti-DAGLA positive patient sera and healthy controls.

Results: IFA screening of sera from P1 to P5 revealed IgG reactivity with the molecular layer in rodent and monkey cerebellum. The dendrites of the purkinje cells are stained while the purkinje cell somata do not react. Furthermore, no IgG reactivity was found with a panel of 30 recombinantly expressed established neural autoantigens. The sera of P1 to P5 immunoprecipitated Sn1-specific diacylglycerol lipase alpha (DAGLA), as detected by Coomassie-stained SDS-PAGE followed by MALDI-TOF mass spectrometry. When the immunoprecipitates were analyzed by Western blot using an monospecific animal antibody against DAGLA, anti-DAGLA showed reactivity with the immunoprecipitate of P1 to P5 while there was no reactivity in the immunoprecipitates of five control sera. However, in the sera of P1 to P5 as well as in seven additional patient sera (P6 to P12) with a similar staining pattern on cerebellum anti-DAGLA antibodies could be detected by RC-IFA with the recombinant protein. Screening of healthy control sera without a specific reaction in IIFT with neuronal tissues (n = 51) revealed no anti-DAGLA positive samples. ELISA using recombinantly expressed fragments of the human DAGLA protein revealed the main reactivity of the anti-DAGLA positive sera with the c-terminal intracellular fragment.

Clinical data from two of the patients with anti-DAGLA autoantibodies were available. P11 suffered from cerebellitis and P12 suffered from epilepsy and hippocampus sclerosis.

### Patients

Control collectives included 51 healthy donors and 40 patients with neurological symptoms and defined anti-neural autoantibodies (3x anti-CASPR2, 5x anti-NMDAR, 5x anti-LGI1, 5x anti-Hu, 2x anti-Hu/anti-Ri 3x anti-Ri, 2x anti-Yo/anti-Ri, 3x anti-Yo, 5x anti-AQP4, 5x anti-GAD65, 2x anti-GABAB receptor).

### Indirect immunofluorescence assay (IFA)

IFA was conducted using slides with a biochip array of brain tissue cryosections (hippocampus of rat, cerebellum of rat and monkey) combined with recombinant HEK293 cells separately expressing 30 different brain antigens Hu, Yo, Ri, CV2, PNMA2, ITPR1, Homer 3, CARP VIII, ARHGAP26, ZIC4, DNER/Tr, GAD65, GAD67, amphiphysin, recoverin, GABA_{B} receptor, glycine receptor, DPPX, IgLON5, glutamate receptors (types NMDA, AMPA, mGluR1, mGluR5, GLURD2), LGl1, CASPR2, AQP4 (M1 and M23), MOG, ATP1A3, NCDN (EUROIMMUN, FA 111a-1003-51, FA 1112-1003-50, FA-1128-1003-50, FA112d-1003-1, FA 112m-1003-50, FA 1151-1003-50, Miske R, Hahn S, Rosenkranz T, Müller M, Dettmann IM, Mindorf S, Denno Y, Brakopp S, Scharf M, Teegen B, Probst C, Melzer N, Meinck HM, Terborg C, Stöcker W, Komorowski L., 2016, Autoantibodies against glutamate receptor δ2 after allogenic stem cell transplantation. Neurol Neuroimmunol Neuroinflamm., 3(4):e255; Scharf M, Miske R, Heidenreich F, Giess R, Landwehr P, Blöcker IM, Begemann N, Denno Y, Tiede S, Dähnrich C, Schlumberger W, Unger M, Teegen B, Stöcker W, Probst C, Komorowski L, 2015, Neuronal Na+/K+ ATPase is an autoantibody target in paraneoplastic neurologic syndrome, Neurology; 84(16):1673-9; Miske R, Gross CC, Scharf M, Golombeck KS, Hartwig M, Bhatia U, Schulte-Mecklenbeck A, Bönte K, Strippel C, Schöls L, Synofzik M, Lohmann H, Dettmann IM, Deppe M, Mindorf S, Warnecke T, Denno Y, Teegen B, Probst C, Brakopp S, Wandinger KP, Wiendl H, Stöcker W, Meuth SG, Komorowski L, Melzer N, 2016, Neurochondrin is a neuronal target antigen in autoimmune cerebellar degeneration, Neurol Neuroimmunol Neuroinflamm.;4(1):e307)). Each biochip mosaic was incubated with 70 µL of PBS-diluted sample at room temperature for 30 min, washed with PBS-Tween and immersed in PBS-Tween for 5 min. In the second step, either Alexa488-labelled goat anti-human IgG (Jackson Research, Suffolk, United Kingdom), or fluorescein isothiocyanate (FITC)-labelled goat anti-human IgG (EUROIMMUN Medizinische Labordiagnostika AG, Lübeck) were applied and incubated at room temperature for 30 min. Slides were washed again with a flush of PBS-Tween and then immersed in PBS-Tween for 5min. Slides were embedded in PBS-buffered, DABCO containing glycerol (approximately 20 µL per field) and examined by fluorescence microscopy. Positive and negative controls were included. Samples were classified as positive or negative based on fluorescence intensity of the transfected cells in direct comparison with non-transfected cells and control samples. Endpoint titers refer to the last dilution showing visible fluorescence.

Results were evaluated by two independent observers using a EUROSTARII microscope (EUROIMMUN Medizinische Labordiagnostika AG, Lübeck, Germany). Reagents were obtained from Merck, Darmstadt, Germany or Sigma-Aldrich, Heidelberg, Germany if not specified otherwise.

### Immunoblot

Immunoprecipitated cerebellum lysate were incubated with NuPage LDS sample buffer (ThermoFisher Scientific, Schwerte, Germany) containing 25 mmol/L dithiothreitol at 70°C for 10 minutes, followed by SDS-PAGE (NuPAGE, ThermoFisher Scientific, Schwerte, Germany). Separated proteins were electrotransferred onto a nitrocellulose membrane by tank blotting with transfer buffer (ThermoFisher Scientific) according to the manufacturer's instructions. The membranes were blocked with Universal Blot Buffer plus (EUROIMMUN Medizinische Labordiagnostika AG, Lübeck) for 15 min and incubated with the patient or control sera (dilution 1:200) or monospecific rabbit antibody against DAGLA (Sigma Aldrich, HPA062497, 1:2,000) in Universal Blot Buffer plus for 3 hours, followed by 3 washing steps with Universal Blot Buffer (EUROIMMUN Medizinische Labordiagnostika AG, Lübeck), a second incubation for 30 min with anti-human-IgG-AP (EUROIMMUN Medizinische Labordiagnostika AG, Lübeck, 1:10) or anti-rabbit-lgG-AP (1:2,000) in Universal Blot Buffer plus, 3 washing steps, and staining with NBT/BCIP substrate (EUROIMMUN Medizinische Labordiagnostika AG, Lübeck). Reagents were obtained from Merck, Darmstadt, Germany or Sigma-Aldrich, Heidelberg, Germany if not specified otherwise.

### Identification of the antigens

Cerebellum from rat was dissected and shock-frozen in liquid nitrogen. The tissues were homogenised in solubilization buffer (100 mmol/L tris-HCI pH 7.4, 150 mmol/L sodium chloride, 2.5 mmol/L ethylenediamine tetraacetic acid, 0.5% (w/v) sodium deoxycholate, 1% (w/v) Triton X-100) containing protease inhibitors (Complete mini, Roche Diagnostics, Penzberg, Germany) with a Miccra D-8 (Roth, Karlsruhe, Germany) and a hand homogenizer (Sartorius, Göttingen, Germany) at 4°C. The tissue lysates was centrifuged at 21,000 x g at 4°C for 15 min and clear supernatants were incubated with patient's serum (diluted 1:16,7) at 4°C overnight. The samples were then incubated with Protein G Dynabeads (ThermoFisher Scientific, Dreieich, Germany) at 4°C for 3 h to capture immunocomplexes. Beads were washed 3 times with PBS, and eluted with NuPage LDS sample buffer (ThermoFisher Scientific, Schwerte, Germany) containing 25 mmol/L dithiothreitol at 70°C for 10 min. Carbamidomethylation with 59 mM iodoacetamide (Bio-Rad, Hamburg, Germany) was performed prior to SDS-PAGE (NuPAGE, ThermoFisher Scientific, Schwerte, Germany). Separated proteins were visualized with Coomassie Brillant Blue (G-250) (Merck), and identified by mass spectrometric analysis.

### Mass spectrometry

Visible protein bands were excised from Coomassie Brilliant Blue G-250 stained gels. After destaining and tryptic digestion peptides were extracted and spotted with α-cyano-4-hydroxycinnamic acid onto a MTP AnchorChip^{™} 384 TF target.

MALDI-TOF/TOF measurements were performed with an Autoflex III smartbeam TOF/TOF200 System using flexControl 3.4 software. MS spectra for peptide mass fingerprinting (PMF) were recorded in positive ion reflector mode with 4,000-10,000 shots and in a mass range from 600 Da to 4,000 Da. Spectra were calibrated externally with the commercially available Peptide Calibration Standard II, processed with flexAnalysis 3.4 and peak lists were analyzed with BioTools 3.2.

The Mascot search engine Mascot Server 2.3 (Matrix Science, London, UK) was used for protein identification by searching against the NCBI or SwissProt database limited to Mammalia. Search parameters were as follows: Mass tolerance was set to 80 ppm, one missed cleavage site was accepted, and carbamidomethylation of cysteine residues as well as oxidation of methionine residues were set as fixed and variable modifications, respectively. To evaluate the protein hits, a significance threshold of p<0.05 was chosen.

For further confirmation of the PMF hits two to five peptides of each identified protein were selected for MS/MS measurements using the WARP feedback mechanism of BioTools. Parent and fragment masses were recorded with 400 and 1000 shots, respectively. Spectra were processed and analyzed as described above with a fragment mass tolerance of 0.7 Da.

### Recombinant expression of DAGLA in HEK293

The cDNA encoding human DAGLA (SEQ ID NO 1) was obtained from Source BioScience UK Limited as clone IRATp970E05140D. By PCR the coding sequence was amplified using the primers sense DAGLA (SEQ ID NO 2) and asense DAGLA (SEQ ID NO 3). The amplification products were digested with BsmBl and ligated with Ncol and Xhol linearized pTriEx-1 (Merck, Darmstadt, Germany). The resulting construct coded SEQ ID NO 4.

DAGLA was transiently expressed in the human cell line HEK293 following PEl-mediated transfection (Exgene 500), according to the manufacturer's instructions (Biomol GmbH, Hamburg, Germany). The cells were harvested 5 days after transfection and lysed by ultrasound. The lysates were stored in aliquots at -80 °C until further use.

### Recombinant expression of DAGLA-Fragments in E. coli

For the expression of DAGLA fragments spanning amino acid residues 1 to 22, 44 to 60, 81 to 101 and 123 to 136 the coding sequence was generated by hybridization of oligodeoxynucleotides sense DAGLA[1-22] and asense DAGLA[1-22] (SEQ ID NO 5 and 6), sense DAGLA[44-60] and asense DAGLA[44-60] (SEQ ID NO 7 and 8), sense DAGLA[81-101] and asense DAGLA[81-101] (SEQ ID NO 9 and 10) and sense DAGLA[123-136] and asense DAGLA[123-136] (SEQ ID NO 11 and 12).

The linker fragments were fused to the octa Histidin (H8) tag and glutathione S-transferase (GST) tag coding sequence by ligation into a modified pET24d (Merck, Darmstadt, Germany) plasmid vector. These constructs encode H8-GST-DAGLA[1-22] (SEQ ID NO 13), H8-GST-DAGLA[44-60] (SEQ ID NO 14), H8-GST-DAGLA[81-101] (SEQ ID NO 15) and H8-GST-DAGLA[123-136] (SEQ ID NO 16) respectively.

The DAGLA[158-598] and DAGLA[583-1042] coding sequence was PCR amplified using human DAGLA cDNA (SEQ ID NO 1) and DNA oligonucleotides sense DAGLA[158-598] and asense DAGLA[158-598] (SEQ ID NO 17 and 18)and sense DAGLA[583-1042] asense DAGLA[583-1042] (SEQ ID NO 19 and 20) respectively. The amplification products were digested with BsmBI and integrated into Ncol Xhol linearized pET24d-N (Exp Dermatol. 2007 Sep;16(9):770-7) encoding H8-DAGLA[158-598] and H8-DAGLA[583-1042] (octa Histidin tag, H8) (SEQ ID NO 21 and 22 respectively).

Bacterial expression was essentially performed as described in the pET system manual (Merck, Darmstadt, Germany) employing E. coli strain RosettaBlue(DE3)pLacl (Merck, Darmstadt, Germany). Protein expression was induced by the addition of 2 mM isopropyl β-D-thiogalactoside (IPTG) for 3 hours at 37°C.

### Purification:

Bacterial cells were lysed, and proteins were solubilized in urea buffer and purified by Ni2+-affinity chromatography following the protocol as in C. Probst, W. Schlumberger, W. Stocker et al., "Development of ELISA for the specific determination of autoantibodies against envoplakin and periplakin in paraneoplastic pemphigus," Clinica Chimica Acta, vol. 410, no. 1-2, pp. 13-18, 2009. The proteins were stored in aliquots at -80 °C until further use.

### Characterization of the patients' autoantibodies

Indirect immunofluorescence assays (IFA) of sera P1 to P5 using permeabilized cryosections of rat and primate cerebellum showed granular staining of the molecular layer (Fig. 1). Further monospecific analyses were conducted with recombinant HEK293 cells expressing 30 neural autoantigens: Hu, Yo, Ri, CV2, PNMA2, SOX1, ITPR1, Homer 3, CARP VIII, ARHGAP26, ZIC4, DNER/Tr, GAD65, GAD67, amphiphysin, recoverin, GABAB receptor, glycine receptor, DPPX, IgLON5, glutamate receptors (types NMDA, AMPA, mGluR1, mGluR5, GLURD2), LGI1, CASPR2, AQP4 (M1 and M23), MOG, ATP1A3 and NCDN. No specific reactivity was observed.

### Identification of DAGLA as the target neuronal autoantigen

The immunoprecipitates from homogenized rat cerebellum obtained with P1 to P5 were subjected to Western Blot analysis. After incubation with the identical serum used for immunoprecipitation specific reactions of approximately 115 kDa were observed which were absent in the immunoprecipitates of the control sera (Fig. 2, A1). In Coomassie-stained SDS-PAGE slight bands could be detected at 115 kDa (Fig. 2, A2). Using MALDI-TOF MS, the protein was identified as DAGLA (UNIPROT acc. # Q5YLM1). As a proof for correct antigen identification, immunoprecipitates were tested by Western blot using antibodies against DAGLA. The immunoprecipitates of the patients' sera contained DAGLA as demonstrated by a 115 kDa band (Fig. 2, A3). Furthermore, the patients' samples were tested by IFA using transfected HEK293 cells which expressed DAGLA (SEQ ID NO 4). Patients' sera reacted with the DAGLA-expressing cells (Fig. 2 B1). In contrast, mock-transfected cell did not demonstrate any specific antibody binding (Fig. 2 B2).

The reaction of the patients' auto-antibodies on tissue could be abolished by pre-incubation with HEK293 lysate containing DAGLA (SEQ ID NO 4) (Fig. 3A). Antibody binding was unaffected when a comparable fraction from mock-transfected HEK293 cells was used.

IIFT with rat and primate cerebellum tissue using patient serum and a specific polyclonal anti-DAGLA antibody (Sigma-Aldrich, Germany) revealed an exact overlap of the molecular layer staining (Fig. 3B).

### Specificity of anti-DAGLA auto-antibodies

Sera from 40 patients with various neural auto-antibody-associated neurological syndromes (3x anti-CASPR2, 5x anti-NMDAR, 5x anti-LGI1, 5x anti-Hu, 2x anti-Hu/anti-Ri 3x anti-Ri, 2x anti-Yo/anti-Ri, 3x anti-Yo, 5x anti-AQP4, 5x anti-GAD65, 2x anti-GABAB receptor), and 51 healthy controls were analyzed by IFA with HEK293-DAGLA in parallel to the samples of the patients. None of the disease control or healthy control sera showed a positive reaction with the HEK293-DAGLA cells.

### Immunoassay for the detection of autoantibodies with DAGLA fragments

Ninety-six-well plates (Nunc, Germany) were coated with 100 µl of the recombinant protein at a concentration of 2,5 µg/ml in PBS for 2 h at 25°C, washed three times with washing buffer (0.05% [wt/vol] Tween 20 in PBS), and then blocked with blocking buffer (0.1% [wt/vol] casein in PBS) for 1 h. The success of antigen immobilization was confirmed by incubation with a murine monoclonal anti-hexahistidine tag antibody (Sigma-Aldrich, Germany) diluted 1:2,000. Experimental serum samples were diluted in sample buffer (1% [wt/vol] casein, 0.05% [wt/vol] Tween 20 in PBS) 1:100 and incubated for 30 min at room temperature. After washing three times, bound antibodies were detected by incubation with anti-mouse IgG-HRP conjugate (Jackson Research, UK) diluted 1:16,000 in sample buffer or anti-human IgG-POD undiluted (Euroimmun, Germany), for 30 min, washed as described above, and incubated with tetramethyl benzidine (TMB) substrate (Euroimmun, Germany) for 15 min. All incubation steps were carried out at room temperature. The optical density (OD) at 450 nm was read using an automated spectrophotometer (Tecan, Germany).

Eight patient sera, which showed a positive reaction in RC-IFA with HEK293-DAGLA and 20 healthy control sera were analyzed by ELISA using six different fragments of the human DAGLA protein (SEQ ID NO 13, 14, 15, 16, 21, 22) (Figure 4). None of the patient sera or control sera showed a positive reaction with the fragments comprising AA 1-22 (SEQ ID NO 23), 44-60 (SEQ ID NO 24), 81-101 (SEQ ID NO 25), and 158-589 (SEQ ID NO 27). Seven patient sera showed a positive reaction with the c-terminal intracellular fragment AA 583-1042 (SEQ ID NO 28) while all of the control sera were negative. Two patient sera additional showed a positive reaction with the extracellular fragment 123-136 (SEQ ID NO 26) which was not observed with the control sera.

## Claims

1. A method for diagnosing a disease comprising the step detecting in a sample from a patient an autoantibody binding to diacylglycerol lipase alpha (DAGLA), wherein the sample is whole blood, plasma, serum or cerebrospinal fluid and wherein the disease is a neurological autoimmune disease associated with an autoantibody binding to DAGLA.

2. An *in vitro* use of a polypeptide comprising diacylglycerol lipase alpha (DAGLA) or a variant thereof for the diagnosis of a disease, wherein the disease is a neurological autoimmune disease associated with an autoantibody binding to DAGLA and wherein the variant is SEQ ID NO26 or SEQ ID NO28.

3. An isolated human autoantibody to diacylglycerol lipase alpha (DAGLA).

4. A complex comprising the autoantibody according to claim 3 and a polypeptide comprising diacylglycerol lipase alpha (DAGLA) or a variant thereof, wherein the variant is SEQ ID NO26 or SEQ ID NO28.

5. A method for isolating a human autoantibody binding to diacylglycerol lipase alpha (DAGLA), comprising the steps
a) contacting a sample comprising the human autoantibody with a polypeptide comprising DAGLA or a variant thereof under conditions compatible with formation of a complex, wherein said human autoantibody binds to said polypeptide,
b) isolating the complex formed in step a),
c1) detecting the complex formed in step a) or c2) dissociating the complex isolated in step b) and separating the autoantibody from the polypeptide,
wherein the sample is whole blood, plasma, serum or cerebrospinal fluid and wherein the variant is SEQ ID NO26 or SEQ ID NO28.

6. A kit for the diagnosis of a disease, which kit comprises a polypeptide comprising diacylglycerol lipase alpha (DAGLA) or a variant thereof or a medical device comprising a polypeptide comprising DAGLA or a variant thereof,
wherein the kit comprises in addition a means for detecting a complex comprising the polypeptide and a human autoantibody binding to DAGLA, wherein said means is a labeled secondary antibody binding to the constant region of the autoantibody and
wherein the disease is a neurological autoimmune disease associated with an autoantibody binding to DAGLA and wherein the variant is SEQ ID NO26 or SEQ ID NO28.

7. The method, use or kit according to any of claims 1, 2, 4, and 6,
wherein the neurological autoimmune disease associated with an autoantibody binding to DAGLA is selected from the group comprising paraneoplastic neurological syndrome (PNS), cerebellitis, epilepsy and sclerosis.

8. The method or use according to any of claims 1, 2, 4 and 7, wherein the autoantibody or complex is detected using a technique selected from the group comprising immunodiffusion techniques, immunoelectrophoretic techniques, light scattering immunoassays, agglutination techniques, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

9. The kit according to any of claims 6 to 8, wherein the medical device is selected from the group comprising a glass slide, preferably for microscopy, a biochip, a microtiter plate, a lateral flow device, a test strip, a membrane, preferably a line blot, a chromatography column and a bead, preferably a magnetic or fluorescent bead.

## Patentansprüche

1. Verfahren zum Diagnostizieren einer Krankheit umfassend den Schritt eines Nachweisens eines Autoantikörpers, der an Diacylglycerol-Lipase alpha (DAGLA) bindet, in einer Probe von einem Patienten, wobei die Probe Vollblut, Plasma, Serum oder Cerebrospinalflüssigkeit ist und wobei die Krankheit eine neurologische Autoimmunkrankheit, die mit einem Autoantikörper, der an DAGLA bindet, assoziiert ist, ist.

2. *In-vitro*-Verwendung eines Polypeptids umfassend Diacylglycerol-Lipase alpha (DAGLA) oder eine Variante davon bei der Diagnose einer Krankheit, wobei die Krankheit eine neurologische Autoimmunkrankheit, die mit einem Autoantikörper, der an DAGLA bindet, assoziiert ist, ist, und wobei die Variante SEQ ID NO26 oder SEQ ID NO28 ist.

3. Isolierter menschlicher Autoantikörper gegen Diacylglycerol-Lipase alpha (DAGLA).

4. Komplex umfassend den Autoantikörper gemäß Anspruch 3 und ein Polypeptid umfassend Diacylglycerol-Lipase alpha (DAGLA) oder eine Variante davon, wobei die Variante SEQ ID NO26 oder SEQ ID NO28 ist.

5. Verfahren zum Isolieren eines menschlichen Autoantikörpers, der an Diacylglycerol-Lipase alpha (DAGLA) bindet, umfassend die Schritte
a) Inkontaktbringen einer Probe, die den menschlichen Autoantikörper umfasst, mit einem Polypeptid umfassend DAGLA oder eine Variante davon unter Bedingungen, die mit einer Bildung eines Komplexes, wobei der menschliche Autoantikörper an das Polypeptid bindet, vereinbar sind,
b) Isolieren des in Schritt a) gebildeten Komplexes,
c1) Nachweisen des in Schritt a) gebildeten Komplexes oder c2) Dissoziieren des in Schritt b) isolierten Komplexes und trennen des Autoantikörpers von dem Polypeptid,
wobei die Probe Vollblut, Plasma, Serum oder Cerebrospinalflüssigkeit ist und wobei die Variante SEQ ID NO26 oder SEQ ID NO28 ist.

6. Kit für die Diagnose einer Krankheit, wobei das Kit ein Polypeptid umfassend Diacylglycerol-Lipase alpha (DAGLA) oder eine Variante davon oder eine medizinische Vorrichtung umfassend ein Polypeptid umfassend DAGLA oder eine Variante davon umfasst,
wobei das Kit zusätzlich ein Mittel zum Nachweisen eines Komplexes umfassend das Polypeptid und einen menschlichen Autoantikörper, der an DAGLA bindet, umfasst, wobei das Mittel ein markierter sekundärer Antikörper, der an die konstante Region des Autoantikörpers bindet, ist und
wobei die Krankheit eine neurologische Autoimmunkrankheit, die mit einem Autoantikörper, der an DAGLA bindet, assoziiert ist, ist und wobei die Variante SEQ ID NO26 oder SEQ ID NO28 ist.

7. Verfahren, Verwendung oder Kit gemäß einem der Ansprüche 1, 2, 4 und 6, wobei die neurologische Autoimmunkrankheit, die mit einem Autoantikörper, der an DAGLA bindet, assoziiert ist, ausgewählt ist aus der Gruppe umfassend paraneoplastisches neurologisches Syndrom (PNS), Cerebellitis, Epilepsie und Sklerose.

8. Verfahren oder Verwendung gemäß einem der Ansprüche 1, 2, 4 und 7, wobei der Autoantikörper oder Komplex unter Verwendung einer Technik nachgewiesen wird, die ausgewählt ist aus der Gruppe umfassend ImmundiBusionstechniken, Immunelectrophorese-Techniken, Lichtstreuungs-Immunassays, Agglutinationstechniken, markierte Immunassays wie jene aus der Gruppe umfassend radiomarkierten Immunassay, Enzym-Immunassays, mehr bevorzugt ELISA, Chemilumineszenz-Immunassays, vorzugsweise Electrochemilumineszenz-Immunassay, und Immunfluoreszenz, vorzugsweise indirekte Immunfluoreszenz.

9. Kit gemäß einem der Ansprüche 6 bis 8, wobei die medizinische Vorrichtung ausgewählt ist aus der Gruppe umfassend einen Glasträger, vorzugsweise für die Mikroskopie, einen Biochip, eine Mikrotiter-Platte, eine Lateral-Flow-Vorrichtung, einen Teststreifen, eine Membran, vorzugsweise einen Linienblot, eine Chromatographie-Säule und ein Bead, vorzugsweise ein magnetisches oder fluoreszierendes Bead.

## Revendications

1. Procédé de diagnostic d'une maladie comprenant l'étape de détection dans un échantillon provenant d'un patient d'un auto-anticorps se liant à la diacylglycérol lipase alpha (DAGLA), dans lequel l'échantillon est du sang entier, du plasma, du sérum ou du liquide céphalo-rachidien et dans lequel la maladie est une maladie auto-immune neurologique associée à un auto-anticorps se liant à DAGLA.

2. Utilisation *in vitro* d'un polypeptide comprenant la diacylglycérol lipase alpha (DAGLA) ou un variant de celle-ci pour le diagnostic d'une maladie, dans laquelle la maladie est une maladie auto-immune neurologique associée à un auto-anticorps se liant à DAGLA et dans laquelle le variant est la SEQ ID NO26 ou la SEQ ID NO28.

3. Auto-anticorps humain isolé contre la diacylglycérol lipase alpha (DAGLA).

4. Complexe comprenant l'auto-anticorps selon la revendication 3 et un polypeptide comprenant la diacylglycérol lipase alpha (DAGLA) ou un variant de celle-ci, dans lequel le variant est la SEQ ID NO26 ou la SEQ ID NO28.

5. Procédé d'isolation d'un auto-anticorps humain se liant à la diacylglycérol lipase alpha (DAGLA), comprenant les étapes
a) mise en contact d'un échantillon comprenant l'auto-anticorps humain avec un polypeptide comprenant DAGLA ou un variant de celle-ci dans des conditions compatibles avec la formation d'un complexe, où ledit auto-anticorps humain se lie audit polypeptide,
b) isolement du complexe formé à l'étape a),
c1) détection du complexe formé à l'étape a) ou c2) dissociation du complexe isolé à l'étape b) et séparation de l'auto-anticorps du polypeptide,
dans lequel l'échantillon est du sang entier, du plasma, du sérum ou du liquide céphalo-rachidien et dans lequel le variant est la SEQ ID NO26 ou la SEQ ID NO28.

6. Kit pour le diagnostic d'une maladie, lequel kit comprend un polypeptide comprenant la diacylglycérol lipase alpha (DAGLA) ou un variant de celle-ci ou un dispositif médical comprenant un polypeptide comprenant DAGLA ou un variant de celle-ci,
dans lequel le kit comprend en outre un moyen de détection d'un complexe comprenant le polypeptide et un auto-anticorps humain se liant à DAGLA, ledit moyen étant un anticorps secondaire marqué se liant à la région constante de l'auto-anticorps et
dans lequel la maladie est une maladie auto-immune neurologique associée à un auto-anticorps se liant à DAGLA et dans lequel le variant est la SEQ ID NO26 ou la SEQ ID NO28.

7. Procédé, utilisation ou kit selon l'une quelconque des revendications 1, 2, 4 et 6,
dans lequel la maladie auto-immune neurologique associée à un auto-anticorps se liant à DAGLA est choisie dans le groupe comprenant le syndrome neurologique paranéoplasique (SNP), la cérébellite, l'épilepsie et la sclérose.

8. Procédé ou utilisation selon l'une quelconque des revendications 1, 2, 4 et 7, dans lequel l'auto-anticorps ou le complexe est détecté en utilisant une technique choisie dans le groupe comprenant les techniques d'immunodiffusion, les techniques d'immunoélectrophorèse, les immunoessais par diffusion de la lumière, les techniques d'agglutination, les immunoessais marqués tels que ceux du groupe comprenant l'immunoessai radiomarqué, les immunoessais enzymatiques, plus préférablement ELISA, les immunoessais par chimiluminescence, de préférence l'immunoessai par électrochimiluminescence, et l'immunofluorescence, de préférence l'immunofluorescence indirecte.

9. Kit selon l'une quelconque des revendications 6 à 8, dans lequel le dispositif médical est choisi dans le groupe comprenant une lame de verre, de préférence pour la microscopie, une biopuce, une plaque de microtitration, un dispositif à flux latéral, une bandelette de test, une membrane, de préférence une membrane à transfert linéaire, une colonne de chromatographie et une bille, de préférence une bille magnétique ou fluorescente.
